# EUROPÄISCHE PATENTSCHRIFT

(11) **EP 0 853 084 B1**
(45) Veröffentlichungstag und Bekanntmachung des Hinweises auf die Patenterteilung: **13.08.2008**
(21) Anmeldenummer: 97121930.8
(22) Anmeldetag: 12.12.1997
(51) Int. Cl.: C07D 473/34, C07D 403/12, C07D 401/12, A61K 31/52

(54) **Substituierte Purinderivate als Vitronectinrezeptor-Antagonisten**
Substituted purine derivatives as vitronectin receptor antagonists
Dérivés de purine substitués comme antagonistes du récpteur de la vitronectine

(30) Priorität: 20.12.1996 DE 19653646
(43) Veröffentlichungstag der Anmeldung: 15.07.1998
(73) Patentinhaber: HOECHST AKTIENGESELLSCHAFT, 65929 Frankfurt am Main (DE); GENENTECH, INC., South San Francisco, CA 94080-4990 (US)
(72) Erfinder: Peyman, Anuschirwan, Dr., 65779 Kelkheim (DE); Knolle, Jochen, Dr., 65830 Kriftel (DE); Wehner, Volkmar, Dr., 97657 Sandberg (DE); Breipohl, Gerhard, Dr., 60529 Frankfurt (DE); Gourvest, Jean-Francois, Dr., 77410 Claye Souilly (FR); Carniato, Denis, Dr., 91460 Marcoussis (FR); Gadek, Thomas Richard, Dr., Oakland, CA 94611 (US)
(74) Vertreter: Vieillefosse, Jean-Claude

(56) Entgegenhaltungen:
- EP-A- 0 531 883
- EP-A- 0 668 278
- WO-A-93/12129
- WO-A-94/15936
- WO-A-96/00730
- WO-A-96/20212
- WO-A-96/40685
- WO-A-96/40709
- JP-A- 47 030 694
- JP-A- 47 030 696
- JP-A- 47 033 196
- JP-A- 63 096 553
- US-A- 5 554 746
- C. G. OVERBERGER ET AL.: "Synthesis of the optically active alpha-nucleic acid base substitutd propanoic acids" TETRAHEDRON LETT., Bd. 30, Nr. 1, 1989, Seiten 51-54, XP002070609
- M. HISATOME ET AL.: "Synthesis and Some Spectroscopic Properties of Porphyrin Derivatives Connected with Nucleobases (Adenine, Thymine, Guanine and Cytosine) by Alkanamide Chains" CHEM. PHARM. BULL., Bd. 44, Nr. 10, 1996, Seiten 1801-1811, XP002070610
- K. A. JACOBSEN ET AL.: "Structure-Activity Relationships of 9-Alkyladenine and Ribose-Modified Adenosine Derivatives at Rat(3) Adenosine Receptors" J. MED. CHEM., Bd. 38, Nr. 10, 1995, Seiten 1720-1735, XP002070611
- N. J. LEONARD, K. ITO: "Controlled Interaction between Nucleic Acid Bases. Intramolecular Stacking Interactions between Two Adenine Rings" J. AMER. CHEM. SOC., Bd. 95, Nr. 12, 1973, Seiten 4010-4016, XP002070612
- P. R. BIRKETT ET AL.: "Synthesis and Intramolecular Cyclisation of 5-Aminoimidazolealkanoates and Their Conversion to Purine Derivatives" SYNTHESIS, Nr. 2, 1991, Seiten 157-159, XP002070613
- T. KANNO, M. KAWAZU: "Studies on the Oxidation of "Reversed Nucleosides" in Oxygen. III. Synthesis of Eritadenine Analogues of Purines and Pyrimidines" CHEM. PHARM. BULL., Bd. 22, 1974, Seiten 2836-2850, XP002070614
- S. PORITERE ET AL.: KHIM. GETEROTSIKL. SOEDIN., Nr. 4, 1982, Seiten 539-541, XP002070615
- S. HILLER ET AL.: KHIM. GETEROTSIKL. SOEDIN., Nr. 12, 1974, Seiten 1680-1683, XP002070616
- S. K. CHAKRABORTI: "Syntheses of Some 9-Substituted Adenines as Inhibitors of Adenosine Deaminase" INDIAN J. CHEM., Bd. 7, Nr. 5, - 1969 XP002070617
- G. ANDREI, E. DE CLERCQ: "Inhibitory effect of selected antiviral compounds on arenavirus replication in vitro" ANTIVIRAL RESEARCH, Bd. 14, Nr. 4-5, 1990, Seiten 287-299, XP002070618
- M.-J. LAN, C. G. OVERBERGER: "Synthesis of Linear Poly(ethylenimine) Containing Nucleic Acid Pendants as Polynucleotide Analogs" J. OLYM. SCI., PART A: POLYM. CHEM., Bd. 25, Nr. 7, 1987, Seiten 1909-1941, XP002070619
- E. V. EFIMTSEVA ET AL.: "Acyclic nucleoside and nucleotide analogues with amide bond" NCLEOSIDES NUCLEOTIDES, Bd. 14, Nr. 3-5, 1995, Seiten 373-375, XP002070620
- N. M. BRAHME, WALTER T. SMITH, JR.: "Some Intramolecular Michael Additions of Adenine Derivatives" J. HETEROCYCL. CHEM., Bd. 22, Nr. 1, 1985, Seiten 109-112, XP002070621
- H. KITANO, H. RINGSDORF: "Surface Behaviors of Nucleic Acid Base-Containing Lipids in Monolayer and Bilayer Systems" BULL. CHEM. SOC. JPN., Bd. 58, Nr. 10, 1985, Seiten 2826-2828, XP002070622
- M. TAKIMOTO ET AL.: "Elementary Patterns in Protein-Nucleic Acid Interactions. II. Crystal Structure of 3-(Adenin-9-yl)propionamide" BULL. CHEM. SOC. JPN., Bd. 54, Nr. 6, 1981, Seiten 1635-1639, XP002070623
- P. R. BIRKETT ET AL.: "Synthesis of Orally Active alpha-(Imidazol-1-yl), Purin-9-yl or Uracil-1-yl) Propanoic and Succinic Acid Derivatives" TETRAHEDRON, Bd. 49, Nr. 47, 1993, Seiten 11029-11036, XP002070624
- M. TAKIMOTO ET AL.: "The Crystal Structure of 3-(Adenin-9-yl)-N-(2-succinimidyl)propiona mide and Hydrogen Bonding Scheme of Anticonvulsant Drugs with Adenine" BULL. CHEM. SOC. JPN., Bd. 57, Nr. 11, 1984, Seiten 3070-3073, XP002070625
- K. OKUMURA ET AL.: "Synthesis and Hypocholesterolemic Activities of Eritadenine Derivatives" J. MED. CHEM., Bd. 17, Nr. 8, 1974, Seiten 846-855, XP002070626
- C. G. OVERBERGER, C. LU: "Chemoselective Acylation for the Synthesis of Model Compounds of Polynucleotide Analogs" J. POLYM. SCI., PART C: POLYM. LETT., Bd. 24, Nr. 6, 1986, Seiten 243-248, XP002070627
- C. G. OVERBERGER, J. I. CHANG: "Synthesis of Optically Active Polynucleotide Analogs with Poly(vinyl Alcohol)s as Backbones and Adenine and 5-Bromouracil Derivatives as Pendants" J. POLYM. SCI., PART A: POLYM. CHEM., Bd. 27, Nr. 11, 1989, Seiten 3589-3602, XP002070628
- C. G. OVERBERGER, C. C. CHEN: "Grafting of Optically Active Nucleic Acid Base Derivatives onto Poly(vinylamine) as Polynucleotide Analogs" J. POLYM. SCI., PART A: POLYM. CHEM., Bd. 25, Nr. 1, 1987, Seiten 389-403, XP002070629
- K. KONDO ET AL.: "Synthesis of Carboxylic Acid Derivatives of Adenine and Theophylline" BULL. CHEM. SOC. JPN., Bd. 44, Nr. 9, 1971, Seiten 2554-2555, XP002070630
- C. G. OVERBERGER, Y. INAKI: "Graft Copolymers Containing Nucleic Acid Bases and L-alpha-Amino Acids" J. POLYM. SCI., POLYM. CHEM. ED., Bd. 17, Nr. 2, 1979, Seiten 1739-1758, XP002070631
- J. A. MONTGOMERY, C. TEMPLE, JR.: "Synthesis of Potential Anticancer Agents. XXVI. The Alkylation of 6-Chloropurine" J. AMER. CHEM. SOC., Bd. 83, 1961, Seiten 630-635, XP002070632

## Beschreibung

Gegenstand der vorliegenden Erfindung sind Verbindungen der Formeln I und Ia, 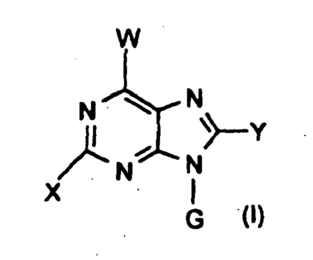 worin X, Y, W, W, G und G^{a} die unten angegebenen Bedeutungen besitzen, sowie ihre physiologisch verträglichen Salze und ihre Prodrugs, ihre Herstellung, ihre Verwendung und sie enthaltende pharmazeutische Präparate.

Die Verbindungen der Formel I sind wertvolle Arzneimittelwirkstoffe. Insbesondere sind sie Vitronectinrezeptor-Antagonisten und eignen sich zur Therapie und Prophylaxe von Krankheiten, die auf der Wechselwirkung zwischen Vitronectinrezeptoren und deren Liganden bei Zell-Zell- oder Zell-Matrix-Interaktionsprozessen beruhen oder die durch eine Beeinflussung dieser Wechselwirkung verhindert, gelindert oder geheilt werden können. Die Erfindung betrifft unter anderem die Verwendung von Verbindungen der Formel I sowie von ihren physiologisch verträglichen Salzen und von pharmazeutischen Präparaten, die solche Verbindungen enthalten, als Heilmittel zur Vorbeugung, Linderung oder Heilung von Krankheiten, die zumindest teilweise durch ein unerwünschtes Maß an Knochenresorption, Angiogenese oder Proliferation von Zellen der glatten Gefäßmuskulatur bedingt sind, oder zu deren Therapie oder Prophylaxe eine Beeinflussung dieser Prozesse angestrebt wird. Insbesondere eignen sich die Verbindungen der Formel I beispielsweise als Inhibitoren der Knochenresorption, als Inhibitoren von Tumorwachstum und Tumormetastasierung, als Entzündungshemmer, zur Behandlung oder Prophylaxe von cardiovaskulären Erkrankungen, wie zum Beispiel Arteriosklerose oder Restenose, oder zur Behandlung oder Prophylaxe von Nephropathien und Retinopathien, wie zum Beispiel diabetischer Retinopathie.

Die ertndungsgemäßen Verbindungen der Formeln I und Ia inhibieren die Knochenresorption durch Osteoclasten. Knochenkrankheiten, gegen die die Verbindungen der Formel I eingesetzt werden können, sind vor allem Osteoporose, Hypercalcämie, Osteopenie, zum Beispiel hervorgerufen durch Metastasen, Zahnerkrankungen, Hyperparathyroidismus, periarticulare Erosionen bei rheumathoider Arthritis und Paget-Krankheit. Ferner können die Verbindungen der Formel I zur Linderung, Vermeidung oder Therapie von Knochenerkrankungen, die durch eine Glucocortikoid-, Steroid- oder Corticosteroid-Therapie oder durch einen Mangel an Sexualhormon(en) hervorgerufen werden, eingesetzt werden. Alle diese Erkrankungen sind durch Knochenverlust gekennzeichnet, der auf dem Ungleichgewicht zwischen Knochenaufbau und Knochenabbau beruht.

Die menschlichen Knochen unterliegen einem fortwährenden dynamischen Umbauprozeß, der Knochenresorption und Knochenaufbau beinhaltet. Diese Prozesse werden von dafür spezialisierten Zelltypen gesteuert. Knochenaufbau beruht auf der Ablagerung von Knochenmatrix durch Osteoblasten, Knochenresorption beruht auf dem Abbau von Knochenmatrix durch Osteoclasten. Die Mehrzahl der Knochenerkrankungen beruhen auf einem gestörten Gleichgewicht zwischen Knochenbildung und Knochenresorption. Osteoporose ist charakterisiert durch einen Verlust an Knochenmatrix. Aktivierte Osteoclasten sind vielkernige Zellen mit einem Durchmesser bis zu 400 µm, die Knochenmatrix abtragen. Aktivierte Osteoclasten lagern sich an die Oberfläche der Knochenmatrix an und sezernieren proteolytische Enzyme und Säuren in die sogenannte "sealing zone", den Bereich zwischen ihrer Zellmembran und der Knochenmatrix. Die saure Umgebung und die Proteasen bewirken den Abbau des Knochens.

Studien haben gezeigt, daß die Anlagerung von Osteoclasten an den Knochen durch Integrin-Rezeptoren auf der Zelloberfläche von Osteoclasten gesteuert wird. Integrine sind eine Superfamilie von Rezeptoren, zu denen unter anderen der Fibrinogenrezeptor a_{IIb}β₃ auf den Blutplättchen und der Vitronectinrezeptor aᵥβ₃ gehören. Der Vitronectinrezeptor aᵥβ₃ ist ein membranständiges Glycoprotein, das auf der Zelloberfläche einer Reihe von Zellen wie Endothelzellen, Zellen der glatten Gefäßmuskulatur, Osteoclasten und Tumorzellen exprimiert wird. Der Vitronectinrezeptor a_{V}β₃, der auf der Osteoclastenmembran exprimiert wird, steuert den Prozeß der Anlagerung an den Knochen und die Knochenresorption und trägt somit zur Osteoporose bei. a_{V}β₃ bindet hierbei an Knochenmatrixproteine wie Osteopontin, Knochensialoprotein und Thrombospontin, die das Tripeptidmotif Arg-Gly-Asp (oder RGD) enthalten.

Horton und Mitarbeiter beschreiben RGD-Peptide und einen anti-Vitronectinrezeptor-Antikörper (23C6), die den Zahnabbau durch Osteoclasten und das Wandern von Osteoclasten inhibieren (Horton et al., Exp. Cell. Res. 1991, 195, 368). Sato et al. beschreiben in J. Cell Biol. 1990, 111, 1713 Echistatin, ein RGD-Peptid aus Schlangengift, als potenten Inhibitor der Knochenresorption in einer Gewebekultur und als Hemmstoff der Osteodasten-Anheftung an den Knochen. Fischer et al. (Endocrinology 1993, 132, 1411) konnten an der Ratte zeigen, daß Echistatin die Knochenresorption auch in vivo hemmt. Wayne et al. (J. Clin. Invest. 1997, 99, 2284) konnten an der Ratte die in vivo-Wirksamkeit der Inhibierung der Knochenresorption durch einen Vitronectinrezeptor-Antagonisten nachweisen.

Der Vitronectinrezeptor a_{V}β₃ auf humanen Zellen der glatten Gefäßmuskulatur der Aorta stimuliert die Wanderung dieser Zellen in das Neointima, was schließlich zu Arteriosklerose und Restenose nach Angioplastie führt (Brown et al., Cardiovascular Res. 1994, 28, 1815).

Brooks et al. (Cell 1994, 79, 1157; J. Clin. Invest. 96 (1995) 1815) sowie Mitjans et al., J. Cell Science 1995, 108, 2825) zeigten, daß Antikörper gegen a_{V}β₃ oder a_{V}β₃-Antagonisten eine Schrumpfung von Tumoren bewirken können, indem sie die Apoptose von Blutgefäßzellen während der Angiogenese induzieren. Cheresh et al. (Science 1995, 270, 1500) beschreiben anti-a_{V}β₃-Antikörper oder a_{V}β₃-Antagonisten, die bFGF-induzierte Angiogeneseprozesse im Rattenauge inhibieren, was sich therapeutisch bei der Behandlung von Retinopathien nutzen läßt.

Aus der EP-A-0 528 586 und der EP-A-0 528 587 sind Aminoalkyl- oder Heterocyclyl-substituierte Phenylalanin-Derivate, aus der WO 95/32710 Arylderivate als Hemmstoffe der Knochenresorption durch Osteoclasten bekannt. In der WO 95/28428 werden RGD-Peptide als Inhibitoren der Knochenresorption, Angiogenese und Restenose beschrieben. In der WO 96/00574 und der WO 96/26190 werden Benzodiazepine unter anderem als Vitronectinrezeptor-Antagonisten bzw. Integrinrezeptor-Antagonisten beschrieben. In der WO 96/00730 werden Fibrinogenrezeptorantagonisten-Template, insbesondere Benzodiazepine, die an einen Stickstoff tragenden 5-Ring geknüpft sind, als Vitronectinrezeptor-Antagonisten, beschrieben. In der EP-A-0 531 883 werden kondensierte 5-gliedrige Heterocyclen beschrieben, die die Fibrinogen-Bindung an Thrombozyten hemmen.

Gegenstand der vorliegenden Erfindung sind Verbindungen der Formeln I und Ia, 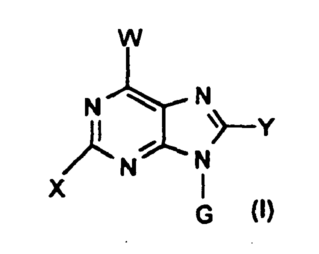 worin bedeuten:
- X: Wasserstoff;
- Y: Wasserstoff;
- G: einen Rest der Formel II

-(CR¹R²)ₙ-A-(CR¹R²)ₘ-(CR¹R³)ᵢ-(CR¹R²)_{q}-R⁴ (II);
- W: einen Rest der Formel III

-8-(CR¹R²)ᵣ₋A'-(CR¹R²)ₛ-(CR¹R³)ₖ-(CR¹R²)ₜ-D-E (III);
- A, A': eine direkte Bindung;
- R¹, R²: unabhängig voneinander Wasserstoff oder (C₁-C₂)-Alkyl, insbesondere Wasserstoff;
- R³: R⁶R^{6'}N-R⁷, R⁶OC(O)N(R⁵)R⁷, R⁶SO₂N(R⁵)R⁷, R⁶C(O)N(R⁵)R⁷ oder R⁶N(R^{6'})C(O)N(R⁵)R⁷, insbesondere R⁶OC(O)N(R⁵)R⁷ ;
- R⁴: C(O)R⁸;
- R⁵: Wasserstoff oder (C₁-C₂)-Alkyl, insbesondere Wasserstoff;
- R⁶, R^{6'}: unabhängig voneinander Wasserstoff, (C₁-C₁₈)-Alkyl, (C₃-C₁₂)-Cycloalkyl, (C₃-C₁₂)-Cycloalkyl-(C₁-C₈)-alkyl, (C₅-C₁₄)-Aryl, worin 1 bis 3 Kohlenstoffatome durch 1 bis 3 Heteroatome aus der Reihe N, S, O ersetzt sein können, oder (C₅-C₁₄)-Aryl-(C₁-C₈)-alkyl, worin in Arylresten 1 bis 3 Kohlenstoffatome durch 1 bis 3 Heteroatome aus der Reihe N, S, O ersetzt sein können, und wobei auch R⁶ und R^{6'} gemeinsam mit den sie verbindenden Atomen ein Ringsystem bilden können, das gegebenenfalls auch zusätzliche, insbesondere ein, zwei oder drei, Heteroatome aus der Reihe N, S, O enthalten kann;
- R⁷: eine direkte Bindung;
- R⁸: Hydroxy, (C₁-C₄)-Alkoxy, (C₅-C₁₄)-Aryl-(C₁-C₄)-alkoxy, (C₅-C₁₄)-Aryloxy, (C₁-C₈)-Alkylcarbonyloxy-(C₁-C₄)-alkoxy oder (C₅-C₁₄)-Aryl-(C₁-C₄)-alkylcarbonyloxy-(C₁-C₄)-alkoxy, insbesondere Hydroxy oder (C₁-C₄)-Alkoxy;
- B: 1,4-Piperidindiyl, wobei das Stickstoffatom des Piperidins an das Puringerüst gebunden ist;
- D: -NR⁶- oder -C(O)-NR⁶-, wobei in der Gruppe -C(O)-NR⁶- das Stickstoffatom an die Gruppe E gebunden ist;
- E: R⁶R^{6'}N-C(=NR^{6'})- oder einen Rest aus der Reihe 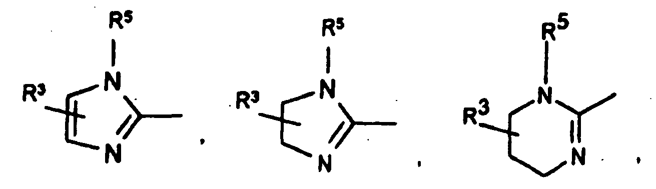 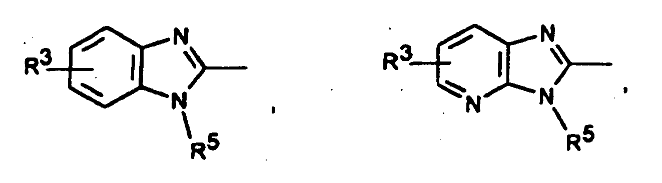
der gegebenenfalls einfach bis dreifach durch Reste aus der Reihe R³, R⁵, =O, =S und R⁶R^{6'}N-C(=NR⁶)- substituiert sein kann;
- r: null oder eins;
- s: null;
- t: null;
- k: null;
- n: eins;
- m: null;
- i: eins;
- q: null;
in allen ihren stereoisomeren Formen und Mischungen davon in allen Verhältnissen, und ihre physiologisch verträglichen Salze.

Speziell bevorzugte erfindungsgemäße Verbindungen sind die Verbindungen der Formel Ih, 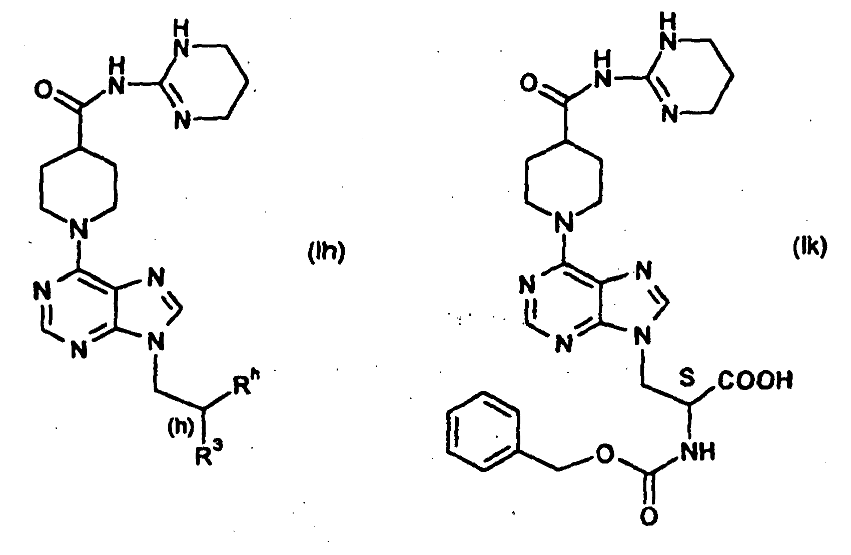 worin R³ für R⁶R^{6'}N-R⁷, R⁶OC(O)N(R⁵)R⁷, R⁶SO₂N(R⁵)R⁷, R⁶C(O)N(R⁵)R⁷ oder R⁶N(R^{6'})C(O)N(R⁵)R⁷, insbesondere für R⁶OC(O)N(R⁵)R⁷, steht und R^{h} für die Carbonsäuregruppe COOH oder für ein Carbonsäurederivat steht, zum Beispiel für einen Ester wie beispielsweise einen (C₁-C₄)-Alkylester, also zum Beispiel für die Gruppe COO-(C₁-C₄)-Alkyl; in allen ihren stereoisomeren Formen und Mischungen davon in allen Verhältnissen, und ihre physiologisch verträglichen Salze sowie ihre Prodrugs. In Verbindungen der Formel Ih, in denen R⁷ für eine direkte Bindung steht, hat bevorzugt das stereochemische Zentrum (h) in der Formel Ih die S-Konfiguration. Verbindungen der Formel Ih, in denen R⁷ für eine direkte Bindung steht, können als an der 2-Aminogruppe gegebenenfalls substituierte 2-Amino-3-(6-(4-(1,4,5,6-tetrahydropyrimidin-2-yl-carbamoyl)-piperidin-1-yl)-purin-9-yl)-propionsäure und Derivate davon, zum Beispiel Ester davon, benannt werden. Besonders speziell bevorzugt ist die 2S-Benzyloxycarbonylamino-3-(6-(4-(1,4,5,6-tetrahydropyrimidin-2-yl-carbamoyl)-piperidin-1-yl)-purin-9-yl)-propionsäure der Formel Ik und ihre physiologisch verträglichen Salze.

Verbindungen der Formeln I können generell, beispielsweise im Zuge einer konvergenten Synthese, durch Verknüpfung zweier oder mehrerer Fragmente, die sich retrosynthetisch aus der Formeln I ableiten lassen, hergestellt werden. Bei der Herstellung der Verbindungen der Formeln I kann es generell im Laufe der Synthese vorteilhaft oder nötig sein, funktionelle Gruppen, die im jeweiligen Syntheseschritt zu unerwünschten Reaktionen oder Nebenreaktionen führen könnten, in Form von Vorstufen einzusetzen, die später in die gewünschten funktionellen Gruppen überführt werden, oder funktionelle Gruppen durch eine dem Syntheseproblem angepaßte Schutzgruppenstrategie temporär zu blockieren, was dem Fachmann bekannt ist (Greene, Wuts, Protective Groups in Organic Synthesis,Wiley, 1991).

Gegenstand der vorliegenden Erfindung sind auch Verfahren zur Synthese der Verbindungen der Formel I, die dadurch gekennzeichnet sind, daß einer oder mehrere der folgenden Schritte zum Aufbau der Verbindungen der Formel I durchgeführt werden.
a1) Eine Verbindung der Formel IV, 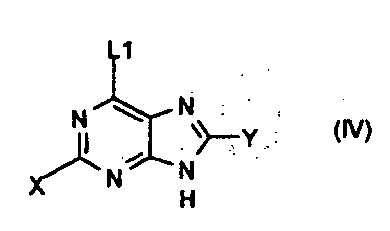 worin
   - L1: für eine übliche, dem Fachmann bekannte Abgangsgruppe, beispielsweise Chlor, Brom, Iod, OTos oder OMes, bevorzugt für Chlor oder Brom steht, und
   - X und Y: wie oben definiert sind, jedoch funktionelle Gruppen gegebenenfalls auch in Form von Vorstufen vorliegen können oder temporär mit einer Schutzgruppe geschützt sein können,
   wird mit einer Verbindung der Formel V,

   L2-(CR¹R²)ₙ-A-(CR¹R²)ₘ-(CR¹R³)ᵢ-(CR¹R²)_{q}-R¹⁰ (V)

   worin
   - R¹, R², R³, A, n, m, i und q: wie oben definiert sind,
   - R¹⁰: wie oben die Gruppe R⁴ definiert ist, jedoch gegebenenfalls mit einer Schutzgruppe geschützt ist, beispielsweise für R⁴ = COOH mit einer tert-Butyl- oder einer Methyl- oder Ethylschutzgruppe,
   - L2: Hydroxy oder eine dem Fachmann bekannte Abgangsgruppe, beispielsweise Chlor, Brom, Iod, OTos, OMes oder OTf bedeutet,
   zu einer Verbindung der Formel VI umgesetzt, 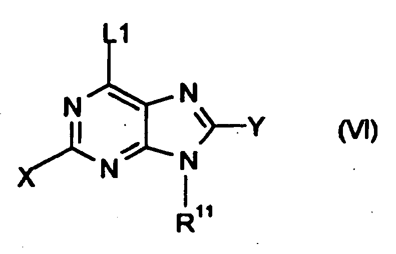 worin
   - R¹¹: für -(CR¹R²)ₙ-A-(CR¹R²)ₘ-(CR¹R³)ᵢ-(CR¹R²)_{q}-R¹⁰ steht und ansonsten die obigen Bedeutungen gelten,
   wobei die Umsetzung nach dem Fachmann bekannten Methoden (s. Quellenliteratur in J. March, Advanced Organic Chemistry, Fourth Edition, Wiley, 1992) erfolgen. Bevorzugt wird in einem geeigneten organischen Lösungsmittel oder Verdünnungsmittel, beispielsweise DCM, CHCl₃, THF, Diethylether, n-Heptan, n-Hexan, n-Pentan, Cyclohexan, Diisopropylether, Methyl-tert-butylether, Acetonitril, DMF, DMSO, Dioxan, Toluol, Benzol, Essigsäureethylester oder einem Gemisch dieser Lösungsmittel, gearbeitet, gegebenenfalls unter Zusatz einer Base wie beispielsweise Butyllithium, Lithiumdiisopropylamid (LDA), Natriumhydrid, Natriumamid, Kalium-tert-butylat, CaCO₃, Cs₂CO₃, Triethylamin, Diisopropylethylamin oder komplexen Basen (Natriumamid/R¹²ONa, wobei R¹² für (C₂-C₆)-Alkyl oder CH₃CH₂OCH₂CH₂ steht). Für L2 = OH kann die Umsetzung beispielsweise nach den für die Mitsunobu-Reaktion beschriebenen Bedingungen erfolgen (Hughes, Organic Reactions 42 (1992) 335-656), beispielsweise durch Umsetzung mit Triphenylphosphin und DEAD in THF.
a2) Die Verbindung der Formel VI wird mit einer Verbindung der Formel VII,

   H-B-(CR¹R²)ᵣ-A'-(CR¹R²)ₛ-(CR¹R³)ₖ-(CR¹R²)ₜ-R¹³ (VII)

   umgesetzt, worin R¹³ für -D-E oder eine Gruppe R¹⁴ steht, die sich in D-E umwandeln läßt und die gegebenenfalls mit geeigneten Schutzgruppen versehen ist, und für die ansonsten die obigen Bedeutungen gelten. R¹⁴ steht beispielsweise für eine gegebenenfalls geschützte Aminogruppe -NHR⁶, wobei als Schutzgruppe beispielsweise die Boc-Schutzgruppe eingesetzt werden kann, einen geschützten Carbonsäureester, einen Aldehyd -C(O)H, eine Ketogruppe -C(O)R⁶, oder eine geschützte Mercaptogruppe.
   Dabei wird eine Verbindung der Formel VIII 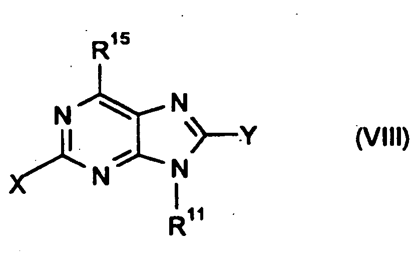 erhalten, worin
   - R¹⁵: für -B-(CR¹R²)ᵣ-A'-(CR¹R²)ₛ-(CR¹R³)ₖ-(CR¹R²)ₜ-R¹³ steht und für die ansonsten die obigen Bedeutungen gelten.

   Die Umsetzung erfolgt nach dem Fachmann bekannten Methoden (s. Quellenliteratur in J. March, Advanced Organic Chemistry, Fourth Edition, Wiley, 1992) bevorzugt in einem geeigneten organischen Lösungsmittel oder Verdünnungsmittel, beispielsweise DCM, CHCl₃, THF, Diethylether, n-Heptan, n-Hexan, n-Pentan, Cyclohexan, Diisopropylether, Methyl-tert-butylether, Acetonitril, DMF , DMSO , Dioxan, Toluol, Benzol, Essigsäureethylester oder Gemischen dieser Lösungsmittel, gegebenenfalls unter Zusatz einer Base wie beispielsweise Butyllithium, Lithiumdiisopropylamid (LDA), Natriumhydrid, Natriumamid, Kalium-tert-butylat, CaCO₃, Cs₂CO₃, Triethylamin, Diisopropylethylamin oder komplexen
   Basen (Natriumamid/R¹²ONa, wobei R¹² für (C₂-C₆)-Alkyl oder CH₃CH₂OCH₂CH₂ steht), wobei für B = NR⁶ auch ein Überschuß an VII als Base dienen kann.
a3) Gegebenenfalls werden die Schutzgruppen in der Verbindung der Formel VIII nach bekannten Methoden (Greene, Wuts, Protective Groups in Organic Synthesis, Wiley, 1991) an R¹³ und/oder R¹⁰ abgespalten. Steht beispielsweise R¹³ für eine mit Boc geschützte Aminogruppe, so kann die Boc-Gruppe beispielsweise durch Umsetzung mit Trifluoressigsäure abgespalten werden.
a4) Danach wird gegebenenfalls R¹³ in der Verbindung der Formel VIII nach bekannten Verfahren zur Gruppe D-E umgesetzt, beispielsweise nach einem der folgenden Verfahren.
   a4.1) Durch Umsetzung von Verbindungen mit R¹³ = NHR⁶ mit 1 H-Pyrazol-1-carboxamidin oder Cyanamid wird ein Guanidin erhalten (siehe Bematowicz et al., J. Org. Chem. 57 (1992) 2497).
   a4.2) Durch Umsetzung von Verbindungen mit R¹³ = NHR⁶ mit einem Monocyclus oder Polycyclus des Typs 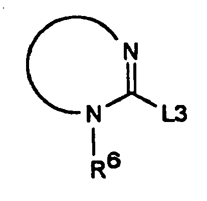 in dem L3 eine nukleophil substituierbare Abgangsgruppe wie zum Beispiel Halogen oder SH, SCH₃, SOCH₃, SO₂CH₃ oder HN-NO₂ darstellt, werden Verbindungen mit der Endgruppe 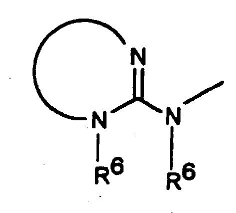 erhalten (zum Verfahren siehe zum Beispiel A.F. Mckay et al., J. Med. Chem. 6 (1963) 587; M.N. Buchman et al., J. Am. Chem. Soc. 71 (1949) 766; F. Jung et al., J. Med. Chem. 34 (1991) 1110; oder G. Sorba et al., Eur. J. Med. Chem. 21 (1986), 391)
   a4.3) Durch Umsetzung von Verbindungen mit R¹³ = NHR⁶ mit Verbindungen des Typs 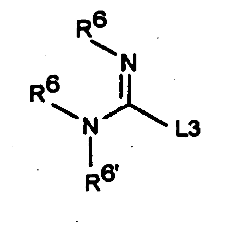 in denen L3 eine nukleophil substituierbare Abgangsgruppe wie zum Beispiel Halogen oder SH, SCH₃, SOCH₃, SO₂CH₃ oder HN-NO₂ darstellt, werden Verbindungen mit der Endgruppe 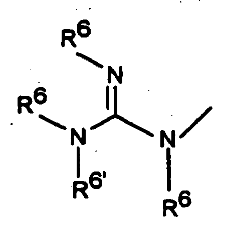 erhalten (zum Verfahren siehe zum Beispiel Miller, Synthesis 1986, 777; oder Brimble, J. Chem. Soc., Perkin Trans. 1 (1990) 311).
   a4.4) Durch Umsetzung von Verbindungen mit R¹³ = NHR⁶ mit einem Monocyclus oder Polycyclus des Typs 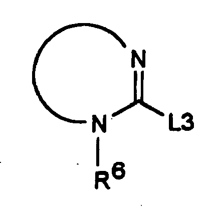 in dem L3 eine nukleophil substituierbare Abgangsgruppe wie zum Beispiel SCH₃ darstellt, werden Verbindungen mit der Endgruppe 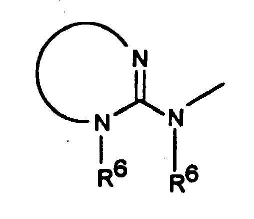 erhalten (zum Verfahren siehe zum Beispiel T. Hiroki et al., Synthesis (1984) 703; oder M. Purkayastha et al., Indian J. Chem. Sect. B 30 (1991) 646).
   a 4.5) Verbindungen, in denen -D-E den Rest eines Aminoguanidinylimins des Typs 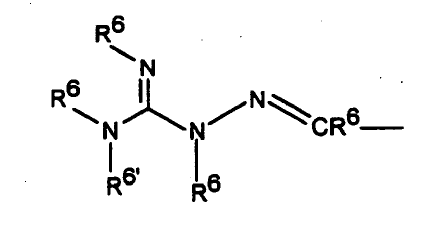 oder eines cyclischen Aminoguanidinylimins des Typs 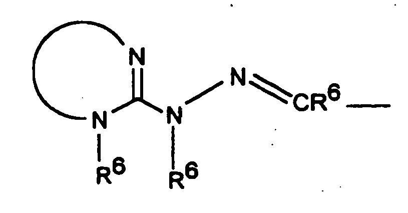 bedeutet, können beispielsweise durch Kondensation von Verbindungen der Formel 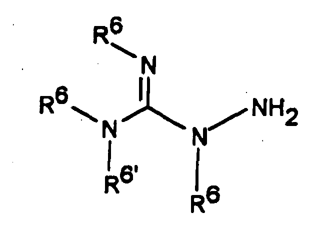 oder 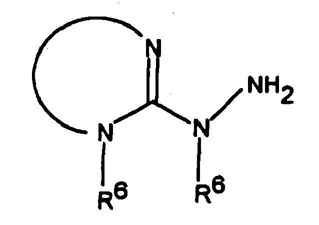 mit Ketonen oder Aldehyden des Typs O=C(R⁶)- oder entsprechenden Acetalen oder Ketalen nach gängigen Literaturverfahren hergestellt werden, beispielsweise analog N. Desideri et al., Arch. Pharm. 325 (1992) 773-777 oder A. Alves et al., Eur. J. Med. Chem. Chim. Ther. 21 (1986) 297-304, wobei obige Aminoguanidinylimine gegebenenfalls als E/Z-Isomerengemische anfallen können, die nach gängigen Chromatographieverfahren getrennt werden können.
   a4.6) Verbindungen, in denen -D-E für R⁶-C(=NR⁶)-NR⁶-N=C(R⁶)- oder für ein einen Monocyclus oder einen Polycyclus enthaltenden Rest des Typs 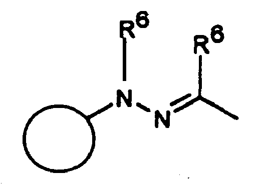 steht, können analog a4.5) erhalten werden.
   a4.7) Verbindungen, in denen -D- für -S(O)₂NR⁶- steht, können beispielsweise hergestellt werden, indem man Verbindungen mit R¹³ = SH nach literaturbekannten Verfahren (vgl. Houben-Weyl, Methoden der Organischen Chemie, Bd. E12/2, Georg Thieme Verlag, Stuttgart 1985, S. 1058ff) zu Sulfonsäuren (R¹³ = SO₃H) oxidiert, aus denen dann zum Beispiel direkt oder über entsprechende Sulfonsäurehalogenide durch Knüpfung einer Amidbindung die Verbindungen mit - D- = -S(O)₂NR⁶- hergestellt werden, wobei oxidationsempfindliche Gruppen im Molekül, wie zum Beispiel Aminogruppen, Amidinogruppen oder Guanidinogruppen, falls erforderlich vor Durchführung der Oxidation durch geeignete Schutzgruppen geschützt werden.
   a4.8) Verbindungen, in denen -D- für -S(O)NR⁶- steht, können beispielsweise hergestellt werden, indem man Verbindungen mit R¹³ = SH in das entsprechende Sulfid überführt und anschließend mit meta-Chlorperbenzoesäure zu den Sulfinsäuren (R¹³ = SO₂H) oxidiert (vgl. Houben-Weyl, Methoden der Organischen Chemie, Bd. E11/1, Georg Thieme Verlag, Stuttgart 1985, S. 618f), aus denen nach literaturbekannten Methoden die entsprechenden Sulfinsäureamide hergestellt werden können. Generell können auch andere literaturbekannte Methoden zur Herstellung von Verbindungen der Formen I und Ia mit -D- = -S(O)ᵤNR⁶- (u = 1, 2) Anwendung finden (vgl. Houben-Weyl, Methoden der Organischen Chemie, Bd. E11/1, Georg Thieme Verlag, Stuttgart 1985, S. 618ff oder Bd. E11/2, Stuttgart 1985, S. 1055ff).
   a4.9) Verbindungen, in denen -D-E für R⁶R^{6'}N-C(=NR⁶)-NR⁶-C(O)- oder den Rest eines cyclischen Acylguanidins des Typs 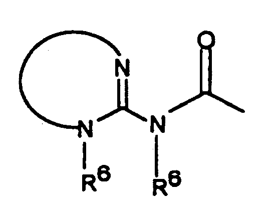 steht, können beispielsweise hergestellt werden, indem man eine Verbindung, in der R¹³ für -C(O)-L4 steht und L4 für eine leicht nukleophil substituierbare Abgangsgruppe steht, mit dem entsprechenden Guanidin(derivat) des Typs 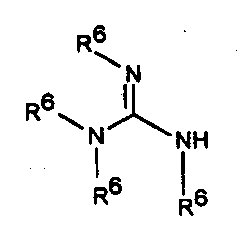 oder dem cyclischen Guanidin(derivat) des Typs 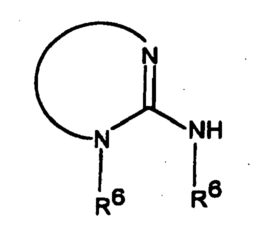 umsetzt. Die vorstehend bezeichneten aktivierten Säurederivate mit der Gruppe L4(O)C-, worin L4 beispielsweise eine Alkoxygruppe, vorzugsweise eine Methoxygruppe, eine Phenoxygruppe, Phenylthiogruppe, Methylthiogruppe, 2-Pyridylthiogruppe oder einen Stickstoffheterocyclus, vorzugsweise 1-Imidazolyl, bedeuten kann, werden vorteilhaft in an sich bekannter Weise aus den zugrundeliegenden Carbonsäurechloriden (L4 = CI) erhalten, die man ihrerseits wiederum in an sich bekannter Weise aus den zugrundeliegenden Carbonsäuren, beispielsweise mit Thionylchlorid, herstellen kann. Neben den Carbonsäurechloriden (L4 = CI) lassen sich auch weitere aktivierte Säurederivate mit der Gruppe Typs L4(O)C- in an sich bekannter Weise direkt aus den zugrundeliegenden Carbonsäuren (L4 = OH) herstellen, wie beispielsweise die Methylester (L4 = OCH₃) durch Behandeln mit gasförmigem HCl in Methanol, die Imidazolide (L4 = 1-Imidazolyl) durch Behandeln mit Carbonyldiimidazol (vgl. Staab, Angew. Chem. Int. Ed. Engl. 1, 351-367 (1962)) oder die gemischten Anhydride (L4 = C₂H₅OC(O)O bzw. TosO) mit Cl-COOC₂H₅ bzw. Tosylchlorid in Gegenwart von Triethylamin in einem inerten Lösungsmittel. Die Aktivierung der Carbonsäuren kann auch mit Carbodiimiden wie Dicyclohexylcarbodiimid (DCCI) oder mit O-((Cyano(ethoxycarbonyl)methylen)amino)-1,1,3,3-tetramethyluronium-tetrafluoroborat ("TOTU") (König et al., Proc. 21st Europ. Peptide Symp. 1990, (Eds. Giralt, Andreu), Escom, Leiden, 1991, S. 143) und anderen in der Peptidchemie gebräuchlichen Aktivierungs-Reagentien erfolgen (eine Reihe geeigneter Methoden zur Herstellung von aktivierten Carbonsäurederivaten sind unter Angabe von Quellenliteratur in J. March, Advanced Organic Chemistry, Third Edition (John Wiley & Sons, 1985), S. 350 angegeben). Die Umsetzung eines aktivierten Carbonsäurederivats mit der Gruppe Typs L4(O)C- mit dem jeweiligen Guanidin(derivat) erfolgt bevorzugt in an sich bekannter Weise in einem protischen oder aprotischen polaren, inerten organischen Lösungsmittel, wobei die Umsetzung der Methylester (L4 = OMe) mit den jeweiligen Guanidinen vorteilhaft in Methanol, Isopropanol oder THF bei Temperaturen von 20°C bis zur Siedetemperatur dieser Lösungsmittel erfolgt. Bei den meisten Umsetzungen von Verbindungen mit der Gruppe L4(O)C- mit salzfreien Guanidinen wird vorteilhaft in aprotischen inerten Lösungsmitteln wie THF, Dimethoxyethan, Dioxan gearbeitet, wobei aber auch Wasser unter Gebrauch einer Base wie beispielsweise NaOH als Lösungsmittel bei der Umsetzung von Verbindungen mit der Gruppe L4(O)C- mit Guanidinen verwendet werden kann. Wenn L4 = CI bedeutet, arbeitet man vorteilhaft unter Zusatz eines Säurefängers, zum Beispiel in Form von überschüssigem Guanidin(derivat), zur Abbindung der Halogenwasserstoffsäure.
   a4.10) Verbindungen, in denen -D-E für R⁶-C(=NR⁶)-NR⁶-C(O)- oder ein einen Monocyclus oder Polycyclus enthaltenden Rest des Typs 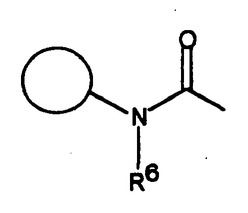 steht, können analog a4.9) erhalten werden.
   a4.11) Verbindungen, in denen -D-E den Rest eines Sulfonylguanidins oder Sulfoxylguanidins des Typs R⁶R^{6'}N-C(=NR⁶)-NR⁶-S(O)ᵤ- (u = 1, 2) bzw. 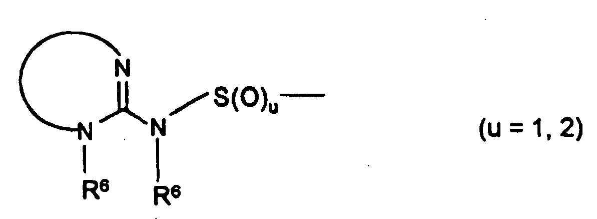 darstellt, können nach literaturbekannten Verfahren durch Reaktion von Verbindungen der Formeln R⁶R^{6'}N-C(=NR⁶)-NHR⁶ bzw. 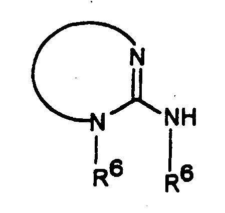 mit Verbindungen, in denen R¹³ für S(O)ᵤ-L5 (u = 1, 2) steht und L5 beispielsweise CI oder NH₂ bedeutet, hergestellt werden, zum Beispiel analog S. Birtwell et al., J. Chem. Soc. (1946) 491 oder Houben Weyl, Methoden der Organischen Chemie, Bd. E4, Georg Thieme Verlag, Stuttgart 1983; S. 620 ff.
   a4.12) Verbindungen, in denen -D-E für R⁶-C(=NR⁶)NR⁶-S(O)ᵤ- (u = 1, 2) oder den einen Monocyclus oder Polycyclus enthaltenden Rest des Typs 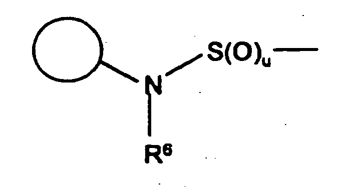 (u= 1, 2) steht, können analog a4.11) erhalten werden.
   a4.13) Verbindungen, in denen -D- für -NR⁶-C(O)- steht, können zum Beispiel hergestellt werden, indem man eine Verbindung mit R¹³ = -NHR⁶ mit einem geeigneten Kohlensäurederivat, bevorzugt Phosgen, Diphosgen (Chlorameisensäure-trichlormethylester), Triphosgen (Kohlensäure-bistrichlormethylester), Chlorameisensäure-ethylester, Chlorameisensäure-i-butylester, Bis-(1-hydroxy-1-H-benzotriazolyl)-carbonat oder N,N'-Carbonyldiimidazol, in einem gegenüber den verwendeten Reagentien inerten Lösungsmittel, bevorzugt DMF, THF oder Toluol, bei Temperaturen zwischen -20°C und dem Siedepunkt des Lösungsmittels, bevorzugt zwischen 0°C und 60°C, zunächst zu einer Verbindung umsetzt, in der R¹³ für 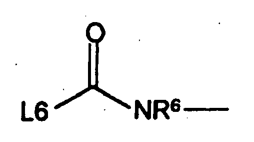 steht, wobei L6 je nach verwendetem Kohlensäurederivat beispielsweise eine Hydroxygruppe, Halogen wie beispielsweise Chlor, Ethoxy, Isobutoxy, Benzotriazol-1-oxy oder 1-Imidazolyl bedeutet. Die anschließende Umsetzung dieser Derivate mit R⁶R^{6'}N-C(=NR⁶)-NR^{6'}H oder R⁶-C(=NR⁶)-NHR⁶ oder mit der einen Monocyclus oder Polycyclus enthaltenden Verbindung des Typs 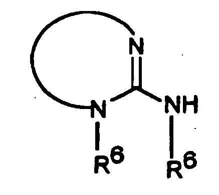 oder 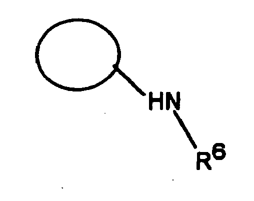 erfolgt dann wie vorstehend für die Herstellung von Acylguanidin(derivat)en in a4.9) beschrieben.
   a4.14) Verbindungen der Formel I, in denen -D-E einen Bis-Aminotriazol-Rest oder einen Bis-Amino-oxadiazol-Rest darstellt, können beispielsweise nach P.J. Garrett et al., Tetrahedron 49 (1993) 165 oder R. Lee Webb et al., J. Heterocyclic Chem. 24 (1987) 275 hergestellt werden.
   a4.15) Verbindungen der Formel I, in denen -D-E eine Hamstoffgruppe oder eine Thioharnstoffgruppe darstellt, können nach bekannten Verfahren synthetisiert werden, wie sie beispielsweise zusammengefaßt sind in C. Ferri, Reaktionen der organischen Synthese, Georg Thieme Verlag, Stuttgart 1978, zum Beispiel durch Umsetzung der entsprechenden Amine mit Isocyanaten bzw. Isothiocyanaten.
a5) Nach der Umsetzung von R¹³ in der Verbindung der Formel VIII zur Gruppe D-E werden gegebenenfalls weitere noch zu entfernende Schutzgruppen nach bekannten Verfahren (siehe Greene, Wuts, s.o.) abgespalten.
a6) Gegebenenfalls werden die erhaltenen Verbindungen der Formel I in ihre Salze, insbesondere in pharmazeutisch verwendbare oder nicht-toxische, physiologisch verträgliche Salze, überführt und/oder in Prodrugs überführt.
   Gegenstand der vorliegenden Erfindung sind weiterhin auch Verfahren zur
   Synthese der Verbindungen der Formel Ia, die dadurch gekennzeichnet sind, daß einer oder mehrere der folgenden Schritte zum Aufbau der Verbindungen der Formel Ia durchgeführt werden.
b1) Eine Verbindung der Formel IV wird mit einer Verbindung der Formel IX,

   L2-(CR¹R²)ᵣ-A'-(CR¹R²)ₛ-(CR¹R³)ₖ-(CR¹R²)ₜ-R¹³ (IX);

   worin R¹, R², R³, A', r, s, k, t, R¹³ und L2 wie oben definiert sind,
   zu einer Verbindung der Formel X umgesetzt, 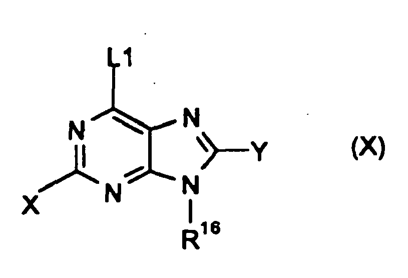 worin R¹⁶ für -(CR¹R²)ᵣ-A'-(CR¹R²)ₛ-(CR¹R³)ₖ-(CR¹R²)ₜR¹³ steht, L1, X und Y wie oben definiert sind und ansonsten die oben angegebenen Bedeutungen gelten. Die Umsetzung erfolgt nach dem Fachmann bekannten Methoden (s. Quellenliteratur in J. March, Advanced Organic Chemistry, Fourth Edition, Wiley, 1992), bevorzugt in einem geeigneten organischen Lösungsmittel oder Verdünnungsmittel, beispielsweise DCM, CHCl₃, THF, Diethylether, n-Heptan, n-Hexan, n-Pentan, Cyclohexan, Diisopropylether, Methyl-tert-butylether, Acetonitril, DMF, DMSO, Dioxan, Toluol, Benzol, Essigsäureethylester oder Gemischen dieser Lösungsmittel, gegebenenfalls unter Zusatz einer Base wie beispielsweise Butyllithium, Lithiumdiisopropylamid (LDA), Natriumhydrid, Natriumamid, Kalium-tert-butylat, CaCO₃, Cs₂CO₃, Triethylamin, Diisopropylethylamin oder komplexen Basen (Natriumamid/R¹²ONa, wobei R¹² für (C₂-C₆)-Alkyl oder CH₃CH₂OCH₂CH₂ steht). Für L2 = OH kann die Umsetzung beispielsweise nach den für die Mitsunobu-Reaktion beschriebenen Bedingungen erfolgen (Hughes, Organic Reactions 42 (1992) 335-656), beispielsweise durch Umsetzung mit Triphenylphosphin und DEAD in THF.
b2) Die Verbindung der Formel X wird mit einer Verbindung der Formel XI,

   H-B-(CR¹R²)ₙ-A-(CR¹R²)ₘ-(CR¹R³)ᵢ-(CR¹R²)_{q}-R¹⁰ (XI);

   in der R¹, R², R³, R¹⁰, A, B, n, m, i und q wie oben definiert sind,
   zu einer Verbindung der Formel XII, 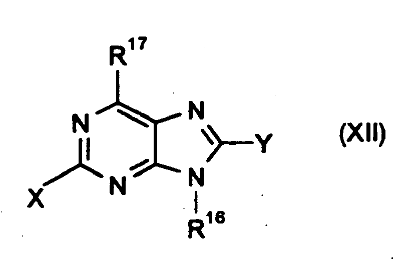 umgesetzt, worin R¹⁶, X und Y wie oben definiert sind, R¹⁷ für -B-(CR¹R²)ₙ-A-(CR¹R²)ₘ-(CR¹R³)ᵢ-(CR¹R²)_{q}-R¹⁰ steht und ansonsten die oben angegebenen Bedeutungen gelten.
b3) Zur weiteren Synthese von Verbindungen der Formel Ia wird anschließend analog den Schritten a3) bis a6) bei der Synthese der Verbindungen der Formel I verfahren.

Im Verfahren zur Synthese der Verbindungen der Formel I kann Schritt a2) auch vor a1) durchgeführt werden. Im Verfahren zur Synthese der Verbindungen der Formel Ia kann Schritt b2) auch vor b1) durchgeführt werden.

Die Einführung von Kohlenstoff-Substituenten in der 6-Position des Purin-Gerüsts kann zum Beispiel durchgeführt werden durch Stille-Kupplung, wie beispielsweise beschrieben in Langli et al., Tetrahedron 52 (1996) 5625; Gundersen, Tetrahedron Lett. 35 (1994) 3153, oder durch Heck-Kopplung, wie beispielsweise beschrieben in Koyama et al., Nucleic Acids Res., Symp. Ser. 11 (1982) 41.

Ein Substituent X in Position 2 des Purin-Gerüsts läßt sich auch am Ende der Synthese der Verbindungen der Formeln I und Ia nach bekannten Verfahren einführen, wie beispielsweise beschrieben in D. A. Nugiel, J. Org. Chem. 62 (1997) 201-203; N. S. Gray, Tetrahedron Lett. 38 (1997) 1161 und der dort zitierten Literatur.

Ein für Y stehende Substituent in der 8-Position läßt sich nach bekannten Verfahren einführen, wie beispielsweise beschrieben in E. J. Reist et al., J. Org. Chem. 33 (1968) 1600; J. L. Kelley et al., J. Med. Chem. 33 (1990) 196; oder E. Vanotti et al., Eur. J. Chem. 29 (1994) 287.

Die erfindungsgemäßen Verbindungen der Formeln I und Ia und ihre physiologisch verträglichen Salze können am Tier, bevorzugt am Säugetier, und insbesondere am Menschen als Arzneimittel für sich allein; in Mischungen untereinander oder in Form von pharmazeutischen Präparaten verabreicht werden, die eine enterale oder parenterale Anwendung gestatten und die als aktiven Bestandteil eine wirksame Dosis mindestens einer Verbindung der Formel I oder der Formel Ia oder eines Salzes davon oder eines Prodrugs davon neben üblichen pharmazeutisch einwandfreien Trägerstoffen und/oder Zusatzstoffen enthalten. Die pharmazeutischen Präparate enthalten normalerweise etwa 0.5 bis 90 Gew.-% der therapeutisch wirksamen Verbindungen.

Die Arzneimittel können oral, zum Beispiel in Form von Pillen, Tabletten, Lacktabletten, Dragees, Granulaten, Hart- und Weichgelatinekapseln, Lösungen, Sirupen, Emulsionen, Suspensionen oder Aerosolmischungen verabreicht werden. Die Verabreichung kann aber auch rektal, zum Beispiel in Form von Suppositorien, oder parenteral, zum Beispiel in Form von Injektionslösungen oder Infusionslösungen, Mikrokapseln oder Rods, perkutan, zum Beispiel in Form von Salben oder Tinkturen, oder nasal, zum Beispiel in Form von Nasalsprays, erfolgen.

Die Herstellung der pharmazeutischen Präparate erfolgt in an sich bekannter Weise, wobei pharmazeutisch inerte anorganische oder organische Trägerstoffe verwendet werden. Für die Herstellung von Pillen, Tabletten, Dragees und Hartgelatinekapseln kann man zum Beispiel Lactose, Maisstärke oder Derivate davon, Talk, Stearinsäure oder deren Salze etc. verwenden. Trägerstoffe für Weichgelatinekapseln und Suppositorien sind zum Beispiel Fette, Wachse, halbfeste und flüssige Polyole, natürliche oder gehärtete Öle etc. Als Trägerstoffe für die Herstellung von Lösungen und Sirupen eignen sich zum Beispiel Wasser, Saccharose, Invertzucker, Glukose, Polyole etc. Als Trägerstoffe für die Herstellung von Injektionslösungen eignen sich Wasser, Alkohole, Glycerin, Polyole, pflanzliche Öle etc. Als Trägerstoffe für Mikrokapseln, Implantate oder Rods eignen sich Mischpolymerisate aus Glykolsäure und Milchsäure.

Die pharmazeutischen Präparate können neben den Wirkstoffen und Trägerstoffen noch Zusatzstoffe, wie zum Beispiel Füllstoffe, Streck-, Spreng-, Binde-, Gleit-, Netz-, Stabilisierungs-, Emulgier-, Konservierungs-, Süß-, Färbe-, Geschmacks-oder Aromatisierungs-, Dickungs-, Verdünnungsmittel, Puffersubstanzen, ferner Lösungsmittel oder Lösungsvermittler oder Mittel zur Erzielung eines Depoteffekts, sowie Salze zur Veränderung des osmotischen Drucks, Überzugsmittel oder Antioxidantien enthalten. Sie können auch zwei oder mehrere Verbindungen der Formeln I oder Ia und/oder deren physiologisch verträgliche Salze enthalten, ferner neben mindestens einer Verbindung der Formeln I oder Ia oder eines Salzes davon noch einen oder mehrere andere therapeutisch wirksame Stoffe.

Die Dosis kann innerhalb weiter Grenzen variieren und ist in jedem einzelnen Fall den individuellen Gegebenheiten anzupassen. Bei oraler Verabreichung beträgt die Tagesdosis im allgemeinen etwa 0.01 bis 100 mg/kg, vorzugsweise 0.1 bis 5 mg/kg, insbesondere 0.3 bis 0.5 mg/kg Körpergewicht zur Erzielung wirksamer Ergebnisse. Auch bei intravenöser Applikation beträgt die Tagesdosis im allgemeinen etwa 0.01 bis 100 mg/kg, vorzugsweise 0.05 bis 10 mg/kg Körpergewicht. Die Tagesdosis kann, insbesondere bei der Applikation größerer Mengen, in mehrere, zum Beispiel 2, 3 oder 4 Teilverabreichungen aufgeteilt werden. Gegebenenfalls kann es, je nach individuellem Verhalten, erforderlich werden, von der angegebenen Tagesdosis nach oben oder nach unten abzuweichen.

Außer als Arzneimittelwirkstoffe können die Verbindungen der Formeln I und Ia auch für diagnostische Zwecke, zum Beispiel in vitro-Diagnosen, und als Hilfsmittel bei biochemischen Untersuchungen eingesetzt werden, bei denen eine Inhibierung des Vitronectinrezeptors oder eine Beeinflussung von Zell-Zell- oder Zell-Matrix-Interaktionen beabsichtigt wird. Weiterhin können sie als Zwischenprodukte für die Herstellung anderer Verbindungen dienen, insbesondere anderer Arzneimittelwirkstoffe, die aus den Verbindungen der Formeln I und la beispielsweise durch Abwandlung oder Einführung von Resten oder Gruppen erhältlich sind.

**Verwendete Abkürzungen:**

| | |
|---|---|
| AcOH | Essigsäure |
| Boc | tert-Butoxycarbonyl |
| DCCl | Dicyclohexylcarbodiimid |
| DCM | Dichlormethan |
| DEAD | Diethylazodicarboxylat |
| DIPEA | Diisopropylethylamin |
| DMF | Dimethylformamid |
| DMSO | Dimethylsulfoxid |
| EE | Ethylacetat |
| HOOBt | 3-Hydroxy-4-oxo-3,4-dihydro-1,2,3-benzotriazin |
| MeOH | Methanol |
| Mes | Methylsulfonyl |
| RT | Raumtemperatur |
| Tf | Trifluormethylsulfonyl |
| THF | Tetrahydrofuran |
| Tos | p-Toluolsulfonyl |
| Z | Benzyloxycarbonyl |

### Beispiele

Verbindungen der Formeln I und Ia, die in der 6-Position des Purin-Gerüsts eine Aminogruppe enthalten, die nicht Bestandteil eines Ringes ist, können auch als Abkömmlinge des Adenins (= 6-Amino-purin) angesehen werden und bei der Benennung der Verbindungen als solche bezeichnet werden. Substituenten, die an 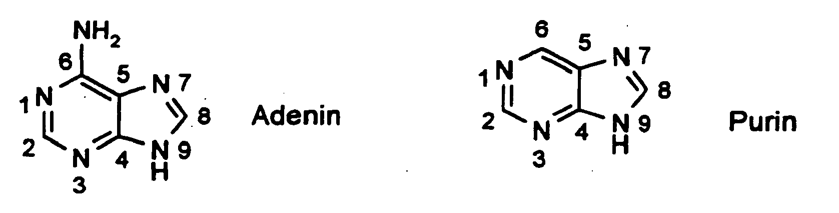 das Stickstoffatom der Aminogruppe in der 6-Position des Adenins gebunden sind, werden bei dieser Bezeichnungsweise mit dem Zusatz N⁶ versehen. Substituenten, die an das Ring-Stickstoffatom in der 9-Position gebunden sind, werden mit dem Zusatz N⁹ versehen. Im Namen des Substituenten wird am Anfang angegeben, über welche Position in dem Substituenten der Substituent bei der gewählten Bezeichnungsweise an das Stickstoffatom N⁶ oder N⁹ gebunden ist. Entsprechendes gilt für Verbindungen, die als N⁹-substituierte Abkömmlinge des Purins bezeichnet werden.

### Beispiel 1

### N⁶-(1-(5-Guanidinopentyl))-N⁹-(3-(2S-(benzyloxycarbonylamino)propionsäure))-adenin

### 1a) N⁹-(3-(2S-(Benzyloxycarbonylamino)-tert-butylpropionat))-6-chlorpurin

2.63 g (17 mmol) 6-Chlorpurin und 4.46 g (16.5 mmol) Triphenylphosphin wurden unter Argon in 50 ml absol. THF suspendiert. Zu dieser Mischung wurden bei RT 2.56 ml (16.3 mmol) DEAD zugegeben und 15 Minuten bei RT gerührt, wobei sich eine klare Lösung bildete. Zu dieser Lösung wurden 3.78 g (12.8 mmol) N-Benzyloxycarbonyl-L-serin-tert-butylester (hergestellt nach M. Schultz, H. Kunz, Tetrahedron: Asymmetry 4 (1993) 1205-1220), gelöst in 50 ml absol. THF, während 1.5 h zugegeben. Danach wurde für weitere 2 h bei RT gerührt. Das Lösungsmittel wurde abgedampft, der Rückstand mit Ether verrieben und über Kieselgel chromatographiert (Toluol:EE 98:2 bis 7:3), wobei 2.85 g (51 %) reines Produkt erhalten wurden.
¹H-NMR (200 MHz, DMSO): δ = 1.30 (s, 9H, C(CH₃)₃); 4.48-4.73 (m, 3H, N⁹-CH₂-CH(NHZ)); 4.98 (s, 2H, CH₂-Aryl); 7.19-7.40 (m, 5H, Aryl-H); 7.87 (d, 1H, NH); 8.61 + 8.77 (2 s, 2H, C⁶-H + C⁸-H).
MS (FAB): m/e = 432.1 (100 %; (M+H)⁺); 376.0 (60).

### 1b) N⁶-(1-(5-(tert-Butyloxycarbonylamino)pentyl))-N⁹-(3-(2S-(benzyloxycarbonylamino)-tert-butylpropionat))-adenin

Zu einer Lösung von 431 mg (1 mmol) N⁹-(3-(2S-(benzyloxycarbonylamino)-tert-butyl-propionat))-chlorpurin (Beispiel 1a) und 404 mg (2 mmol) 5-(tert-Butyloxycarbonylamino)-1-pentylamin in 5 ml absol. DMF wurden 0.170 ml (1 mmol) DIPEA und 5 mg Kaliumiodid zugegeben und die Mischung 72 h bei 40 °C gerührt. Das Lösungsmittel wurde abgedampft und der Rückstand über Kieselgel chromatographiert (Toluol:EE 7:3 bis 1:2), wobei 190 mg (32 %) reines Produkt erhalten wurden.
MS (FAB): m/e = 598.3 (100 %; (M+H)⁺).

### 1c) N⁶-(1-(5-Aminopentyl))-N⁹-(3-(2-(benzyloxycarbonylamino)propionsäure))-adenin

190 mg (0.32 mmol) N⁶-(1-(5-(tert-Butyloxycarbonylamino)pentyl))-N⁹-(3-(2S-(benzyloxycarbonylamino)-tert-butylpropionat))-adenin (Beispiel 1 b) wurden in 2 ml 90 %iger Trifluoressigsäure gelöst und 2 h bei RT gerührt. Es wurde zur Trockne eingedampft und der Rückstand zweimal mit Essigsäure koevaporiert. Danach wurde in Wasser aufgelöst und gefriergetrocknet. Ausbeute: 134 mg (95 %).
MS (ES+): m/e = 442.3 (20 %; (M+H)⁺), 308.2 (35).

### 1d) N⁶-(1-(5-Guanidinopentyl))-N⁹-(3-(2S-(benzyloxycarbonylamino)propionsäure))-adenin

34 mg (0.077 mmol) N⁶-(1-(5-Aminopentyl))-N⁹-(3-(2S-(benzyloxycarbonylamino)-propionsäure))-adenin (Beispiel 1 c) wurde in 1.5 ml Wasser und 0.5 ml DMF gelöst und mit 0.033 ml (0.193 mmol) DIPEA und 13.5 mg (0.092 mmol) 1H-Pyrazol-1-carboxamidin-Hydrochlorid versetzt und 40 h bei RT gerührt. Danach wurde das Lösungsmittel abgedampft, der Rückstand in Wasser aufgenommen und gefriergetrocknet. Zur weiteren Reinigung wurde über Kieselgel chromatographiert (DCM:Methanol:Essigsäure:Wasser 15:5:1:1). Ausbeute: 70 %.
MS (FAB): m/e = 484.2 (100 %; (M+H)⁺).

### Beispiel 2

### N⁶-(1-(4-Guanidinobutyl))-N⁹-(3-(2S-(benzyloxycarbonylamino)propionsäure))-adenin

### 2a) N⁶-(1-(4-(tert-Butyloxycarbonylamino)butyl))-N⁹-(3-(2S-(benzyloxycarbonylamino)-tert-butylpropionat))-adenin

Synthese analog 1b aus 431 mg (1 mmol) N⁹-(3-(2S-(Benzyloxycarbonylamino)-tert-butyl-propionat))-6-chlorpurin (Beispiel 1a) und 376 mg (2 mmol) 4-(tert-Butyloxycarbonylamino)-1-butylamin. Ausbeute: 214 mg (37 %).
¹H-NMR (200 MHz, DMSO): δ = 1.30 (s, 9H, C(CH₃)₃); 1.38 (s, 9H, C(CH₃)₃); 1.41 (m, 2H, CH₂); 1.57(m, 2H, CH₂); 3.46 (m, 2H, CH₂-NH-Boc); 2.92 (t, 2H, C²-NH-CH₂); 4.31-4.58 (m, 3H, N¹-CH₂-CH(NHZ)); 5.01 (s, 2H, CH₂-Aryl); 6.99 (t, 1H, C²-NH); 7.10-7.38 (m, 5H, Aryl-H); 7.75 (m, 1H, NH-Boc); 7.91 (d, 1H, NH-Z); 8.02 + 8.20 (2 s, 2H, C⁶-H + C⁸-H).
MS (ES+): m/e = 584.3 (100 %; (M+H)⁺).

### 2b) N⁶-(1-(4-Aminobutyl))-N⁹-(3-(2S-(benzyloxycarbonylamino)propionsäure))-adenin

Synthese analog Beispiel 1c aus N⁶-(1-(4-tert-Butyloxycarbonylamino)butyl)-N⁹-(3-(2S-(benzyloxycarbonylamino)-tert-butylpropionat))-adenin (Beispiel 2a). Ausbeute: 96 %.
MS (ES+): m/e = 428.2 (100 %; (M+H)⁺), 294.1 (70).

### 2c) N⁶-(1-(4-Guanidihobutyl))-N⁹-(3-(2S-(benzyloxycarbonylamino)propionsäure))-adenin

Synthese analog Beispiel 1d aus N⁶-(1-(4-Aminobutyl))-N⁹-(3-(2S-(benzyloxycarbonylamino)propionsäure))-adenin (Beispiel 2b). Ausbeute: 76 %.
MS (ES+): m/e = 470.1 (20 %; (M+H)⁺).

### Beispiel 3

### N⁶-(1-(3-Guanidinopropyl))-N⁹-(3-(2S-(benzyloxycarbonylamino)propionsäure))-adenin

### 3a) N⁶-(1-(3-(tert-Butyloxycarbonylamino)propyl))-N⁹-(3-(2S-(benzyloxycarbonylamino)-tert-butylpropionat))-adenin

Synthese analog 1 b aus 60 mg (0.14 mmol) N⁹-(3-(2S-(benzyloxycarbonylamino)-tert-butyl-propionat))-6-chlorpurin (Beispiel 1 a) und 30 mg (0.17 mmol) 3-(tert-Butyloxycarbonylamino)-1-propylamin. Ausbeute: 30 mg (38 %).
¹H-NMR (200 MHz, DMSO): δ = 1.28 (s, 9H, C(CH₃)₃); 1.36 (s, 9H, C(CH₃)₃); 1.68 (m, 2H, CH₂-CH₂-CH₂); 1.41 (m, 2H, CH₂); 2.98 (t, 2H, C²-NH-CH₂); 3.46 (t, 2H, CH₂-NH-Boc); 4.29-4.59 (m, 3H, N¹-CH₂-CH(NHZ)); 5.00 (s, 2H, CH₂-Aryl); 6.82 (t, 1H, C²-NH); 7.21-7.40 (m, 5H, Aryl-H); 7.72 (m, 1H, NH-Boc); 7.91 (d, 1H, NH-Z); 8.03 + 8.20 (2 s, 2H, C⁶-H + C⁸-H).
MS (ES+): m/e = 570.3 (100 %; (M+H)⁺).

### 3b) N⁶-(1-(3-Aminopropyl))-N⁹-(3-(2S-(benzyloxycarbonylamino)propionsäure))-adenin

Synthese analog Beispiel 1 c aus N⁶-(1-(3-(tert-Butyloxycarbonylamino)propyl))-N⁹-(3-(2S-(benzyloxycarbonylamino)-tert-butylpropionat))-adenin (Beispiel 3a).
Ausbeute: 100 %.
MS (ES+): m/e = 414.2 (100 %; (M+H)⁺), 280.1 (30).

### 3c) N⁶-(1-(3-Guanidinopropyl))-N⁹-(3-(2S-(benzyloxycarbonylamino)propionsäure))-adenin

Synthese analog Beispiel 1d aus N⁶-(1-(3-Aminopropyl))-N⁹-(3-(2S-(benzyloxycarbonylamino)propionsäure))-adenin (Beispiel 3b). Ausbeute: 66 %.
MS (ES+): m/e = 456.3 (20 %; (M+H)⁺), 130.1 (100).

### Beispiel 4

### N⁶-(1-(4-(4,5-Dihydro-1H-imidazol-2-ylamino)butyl))-N⁹-(3-(2S-(benzyloxycarbonylamino)propionsäure))-adenin

153 mg (0.36 mmol) N⁶-(1-(4-aminobutyl))-N⁹-(3-(2S-(benzyloxycarbonylamino)propionsäure))-adenin (Beispiel 2b) und 88 mg (0.36 mmol) 2-(Methylmercapto)-2-imidazolin-Hydroiodid wurden in 2 ml Wasser gelöst und mit 1 N NaOH auf pH 9.0 eingestellt. Es wurde 100 h bei 50°C gerührt. Danach wurde die Lösung mit 1 N HCl auf pH 1.5 gebracht, das Lösungsmittel abgedampft und der Rückstand mehrfach über Kieselgel chromatographiert (DCM:MeOH 9:1 bis 1:2, jeweils mit 0.1% AcOH, 0.1 % H₂O), dann DCM:MeOH:H₂O:AcOH 8:2:0.4:0.4. Ausbeute: 7 mg (4 %).
MS (FAB): m/e = 496.2 (M+H⁺, 100 %); 518.2 (M+Na⁺, 50).

### Beispiel 5

### N⁶⁻(1-(3-Guanidinopropyl))-N⁹-(4-(2S-(benzyloxycarbonylamino)buttersäure))-adenin

### 5a) N⁹-(4-(2S-(benzyloxycarbonylamino)buttersäure-tert-butylester))-6-chlorpurin

Synthese analog Beispiel 1a aus 6-Chlorpurin und N-Benzyloxycarbonyl-L-homoserin-tert-butylester. Ausbeute: 24 %.
¹H-NMR (200 MHz, DMSO): δ = 1.34 (s, 9H, C(CH₃)₃); 2.08-2.43 (m, 2H, N-CH₂-CH₂-CH); 3.81-3.93(m, 1H, CH-NHZ); 4.39 (t, 2H, N⁹-CH₂); 5.02 (s, 2H, CH₂-Aryl); 7.26-7.42 (m, 5H, Aryl-H); 7.87 (d, 1H, NH); 8.63 + 8.75 (2 s, 2H, C⁶-H + C⁸-H).
MS (FAB): m/e = 446.1 (100 %; (M+H)⁺); 390.1 (65).

### 5b) N⁶-(1-(3-(tert-Butyloxycarbonylamino)propyl))-N⁹-(4-(2S-(benzyloxycarbonylamino)buttersäure-tert-butylester))-adenin

Synthese analog 1 b aus 50 mg (0.11 mmol) N⁹-(4-(2S-(benzyloxycarbonylamino)buttersäure-tert-butylester))-6-chlorpurin (Beispiel 5a) und 38 mg (0.22 mmol) 3- (tert-Butyloxycarbonylamino)-1-propylamin. Ausbeute: 26 mg (41 %).
MS (ES+): m/e = 584.3 (100 %; (M+H)⁺).

### 5c) N⁶-(1-(3-Aminopropyl))-N⁹-(4-(2-(benzyloxycarbonylamino)buttersäure))-adenin

Synthese analog Beispiel 1c aus N⁶-(1-(3-(tert-Butyloxycarbonylamino)propyl))-N⁹-(4-(2S-(benzyloxycarbonylamino)buttersäure-tert-butylester))-adenin (Beispiel 5b). Ausbeute: 94 %.
MS (FAB): m/e = 428.3 (100 %; (M+H)⁺).

### 5d) N⁶-(1-(3-Guanidinopropyl))-N⁹-(4-(2S-(benzyloxycarbonylamino)buttersäure))-adenin

Synthese analog Beispiel 1d aus N⁶-(1-(3-Aminopropyl))-N⁹-(3-(2S-(benzyloxycarbonylamino)buttersäure))-adenin (Beispiel 5c). Ausbeute: 71 %.
MS (FAB): m/e = 470.3 (70 %; (M+H)⁺).

### 5e) N-Benzyloxycarbonyl-L-homoserin

6 g (50.4 mmol) L-Homoserin wurden in 50 ml DMF weitgehend gelöst und bei 0°C portionsweise mit 12.56 g (50.4 mmol) N-(Benzyloxycarbonyloxy)-succinimid versetzt. Es wurde 1 h bei 0°C, dann 48 h bei RT gerührt. Das Lösungsmittel wurde abdestilliert und der Rückstand zwischen EE und einer gesättigten NaCl-Lösung verteilt. Die organische Phase wurde mit gesättigter NaCl-Lösung, mit 5 %iger Citronensäure und nochmal mit gesättigter NaCl-Lösung gewaschen, getrocknet, filtriert und eingeengt. Der kristalline Rückstand wurde in Ether verrührt, abgesaugt, mit Ether und Pentan gewaschen. Ausbeute: 9.55 g (75 %).
¹H-NMR (200 MHz, DMSO): δ = 1.61-1.95 (m, 2H, CH₂-CH₂-OH); 3.42 (m, 2H, CH₂-OH); 4.08 (m, 1H, CH-NH-Z); 4.57 (s, breit, 1H, OH); 5.02 (s, 2H, CH₂-Ph); 7.32 (m, 5H, Aryl-H), 7.49 (d, 1 H, NH-Z).
MS (CI+): m/e = 236.1 (M+H⁺-H₂O, 20 %); 192.1 (50); 91.0 (100).

### 5f) N-Benzyloxycarbonyl-L-homoserin-tert-butylester

3.8 g (15 mmol) Z-L-Homoserin und 3.42 g (15 mmol) Benzyltriethylammoniumchlorid wurden unter Argon in 110 ml N-Methyl-2-pyrrolidon gelöst und nacheinander mit 53.9 g (390 mmol). K₂CO₃ und 98.7 g (720 mmol) tert-Butylbromid versetzt. Es wurde 22 h bei 55 °C gerührt. Das Reaktionsgemisch wurde in 1.5 l Eiswasser gegossen, zweimal mit Toluol extrahiert, die organische Phase zweimal mit gesättigter NaCl-Lösung gewaschen, getrocknet, filtriert und eingeengt. Das Produkt wurde zur weiteren Reinigung über Kieselgel chromatographiert (n-Heptan : EE 7:3 bis 1:1). Ausbeute: 2.0 g (43.1 %).
¹H-NMR (200 MHz, CDCl₃): δ = 1.45 (s, 9H, tBu); 1.51-1.74 + 2.03-2.26 (m, 2H, CH₂-CH₂-OH); 3.01 (s, breit, 1H, OH); 3.70 (m, 2H, CH₂-OH); 4.41 (m, 1H, CH-NH-Z); 5.12 (s, 2H, CH₂-Ph); 5.60 (d, 1 H, NH-Z); 7.36 (m, 5H, Aryl-H),
MS (CI+): m/e = 310.3 (M+H⁺, 50 %); 254.2 (100).

### Beispiel 6

### N⁶-(1-(4-Guanidinobutyl))-N⁹-(4-(2S-(benzyloxycarbonylamino)buttersäure))-adenin

### 6a) N⁶-(1-(4-(tert-Butyloxycarbonylamino)butyl)-N⁹-(4-(2S-(benzyloxycarbonylamino)buttersäure-tert-butylester))-adenin

Synthese analog 1 b aus 50 mg (0.11 mmol) N⁹-(4-(2S-(benzyloxycarbonylamino)buttersäure-tert-butylester))-6-chlorpurin (Beispiel 5a) und 41 mg (0.22 mmol) 4-(tert-Butyloxycarbonylamino)-1-butylamin. Ausbeute: 38 mg (58%).
MS (ES+): m/e = 598.3 (100 %; (M+H)⁺).

### 6b) N⁶-(1-(4-Aminobutyl))-N⁹-(4-(2-(benzyloxycarbonyl-amino)buttersäure))-adenin

Synthese analog Beispiel 1c aus N⁶-(1-(4-(tert-Butyloxycarbonylamino)butyl))-N⁹-(4-(2S-(benzyloxycarbonylamino)buttersäure-tert-butylester))-adenin (Beispiel 6a). Ausbeute: 100 %.
MS (FAB): m/e = 442.3 (100 %; (M+H)⁺).

### 6c) N⁶-(1-(4-Guanidinobutyl))-N⁹-(4-(2S-(benzyloxycarbonylamino)buttersäure))-adenin

Synthese analog Beispiel 1d aus N⁶-(1-(4-Aminobutyl))-N⁹-(3-(2S-(benzyloxycarbonylamino)buttersäure))-adenin (Beispiel 6b). Ausbeute: 65 %.
MS (ES+): m/e = 484.3 (5 %; (M+H)⁺), 350.2 (10), 333.2 (5), 130.0 (100).

### Beispiel 7

### N⁶-(1-(3-Guanidinopropyl))-N⁹-(3-propionsäure)-adenin

### 7a) N⁹-(3-Propionsäure-tert-butylester)-6-chlorpurin

15.45 g (0.1 mol) 6-Chlorpurin, 43.5 ml (0.3 mol) tert-Butylacrylat und 1.34 ml (7 mmol) 5.22 N Natriummethanolat (in MeOH) wurden in 400 ml absol. MeOH gelöst und unter nochmaligem Zusatz von 2.6 ml (14 mmol) 5.22 N Natriummethanolat (in MeOH) 4.5 h unter Rückfluß gekocht. Zur Aufarbeitung wurde abgesaugt, das Lösungsmittel abgedampft und der Rückstand über Kieselgel (+10 % H₂O) chromatographiert (Toluol:EE 3:1). Ausbeute: 1.35 g (5 %).
¹H-NMR (200 MHz, DMSO): δ = 1.29 (s, 9H, C(CH₃)₃); 2.95 (t, 2H, CH₂C(O)); 4.50 (t, 2H, N-CH₂); 8.70 + 8.79 (2 s, 2H, C⁶-H + C⁸-H).
MS (ES+): m/e = 283.1 (70 %; (M+H)⁺); 227.0 (100).

### 7b) N⁶-(1-(3-(tert-Butyloxycarbonylamino)propyl))-N⁹-(3-propionsäure-tert-butylester)-adenin

Synthese analog 1 b aus 282 mg (1.0 mmol) N⁹-(3-Propionsäure-tert-butylester)-6-chlorpurin (Beispiel 7a) und 209 mg (1.2 mmol) 3-(tert-Butyloxycarbonylamino)-1-propylamin. Ausbeute: 160 mg (38 %).
MS (ES+): m/e = 421.2 (100 %; (M+H)⁺), 365.2 (60), 321.2 (50), 265.1 (30).

### 7c) N⁶-(1-(3-Aminopropyl))-N⁹-(3-propionsäure)-adenin

Synthese analog Beispiel 1c aus N⁶-(1-(3-(tert-Butyloxycarbonylamino)propyl))-N⁹-(3-propionsäure-tert-butylester)-adenin (Beispiel 7b). Ausbeute: 100 %.
¹H-NMR (200 MHz, DMSO): δ = 1.88 (t, 2H, CH₂-CH₂-CH₂-); 2.80-2.93 (m, 4H, NH-CH₂ + CH₂-C(O)); 3.56 (m, 2H, CH₂-NH₂); 4.38 (t, 2H, N⁹-CH₂); 7.72 (s, breit, 2H, NH₂); 7.95 (t, 1H, NH); 8.15 + 8.23 (2 s, 2H, C⁶-H + C⁸-H).
MS (ES+): m/e = 265.1 (100 %; (M+H)⁺); 248.1 (40), 176.0 (30).

### 7d) N⁶-(1-(3-Guanidinopropyl))-N⁹-(3-propionsäure)-adenin

Synthese analog Beispiel 1d aus N⁶-(1-(3-Aminopropyl))-N⁹-(3-propionsäure)-adenin (Beispiel 7c). Ausbeute: 41 %.
¹H-NMR (200 MHz, D₂O): δ = 1.95 (t, 2H, CH₂-CH₂-CH₂-); 2.71 (t, 2H, CH₂-C(O)); 3.24 (t, 2H, Gua-CH₂); 3.65 (m, 2H, CH₂-NH₂); 4.40 (t, 2H, N⁹-CH₂); 8.00 + 8.15 (2 s, 2H, C⁶-H + C⁸-H).
MS (ES+): m/e = 307.1 (100 %; (M+H)⁺), 290.1 (30).

### Beispiel 8

### N⁶-(1-(4-Guanidinobutyl))-N⁹-(3-propionsäure)-adenin

### 8a) N⁶-(1-(4-(tert-Butyloxycarbonylamino)butyl))-N⁹-(3-propionsäure-tert-butylester)-adenin

Synthese analog 1b aus 141 mg (0.5 mmol) N⁹-(3-Propionsäure-tert-butylester)-6-chlorpurin (Beispiel 7a) und 104 mg (0.55 mmol) 4-(tert-Butyloxycarbonylamino)-1-butylamin. Ausbeute: 130 mg (60 %).
¹H-NMR (200 MHz, DMSO): δ = 1.32 (s, 9H, C(CH₃)₃); 1.35 (s, 9H, C(CH₃)₃); 1.40 (t, 2H, CH₂); 1.57 (t, 2H, CH₂); 2.84 (t, 2H, -CH₂-C(O)); 2.95 (t, 2H, C²-NH-CH₂); 3.45 (m, 2H, CH₂-NH-Boc); 4.34 (t, 2H, N⁹-CH₂); 6.78 (t, 1H, C²-NH); 7.70 (m, 1H, NH-Boc); 8.08 + 8.19 (2 s, 2H, C⁶-H + C⁸-H).
MS (ES+): m/e = 435.2 (100 %; (M+H)⁺), 379.2 (20), 335.2 (55), 279.1 (50).

### 8b) N⁶-(1-(4-Aminobutyl))-N⁹-(3-propionsäure)-adenin

Synthese analog Beispiel 1c aus N⁶-(1-(4-(tert-Butyloxycarbonylamino)butyl)-N⁹-(3-propionsäure-tert-butylester)-adenin (Beispiel 8a). Ausbeute: 100 %.
¹H-NMR (200 MHz, DMSO): δ = 1.50-1.70 (m, 4H,-CH₂-CH₂-); 2.74-2.91 (m, 4H, NH-CH₂ + CH₂-C(O)); 3.50 (m, 2H, CH₂-NH₂); 4.36 (t, 2H, N⁹-CH₂); 7.64 (s, breit, 2H, NH₂); 7.90 (t, 1 H, NH); 8.11 + 8.21 (2 s, 2H, C⁶-H + C⁸-H).
MS (FAB): m/e = 279.2 (100 %; (M+H)⁺).

### 8c) N⁶-(1-(4-Guanidinobutyl))-N⁹-(3-propionsäure)-adenin

Synthese analog Beispiel 1d aus N⁶-(1-(4-Aminobutyl))-N⁹-(3-propionsäure)-adenin (Beispiel 8b). Ausbeute: 65 %.
MS (ES+): m/e = 321.1 (100 %; (M+H)⁺).

### Beispiel 9

### N⁶-(1-(5-Guanidinopentyl))-N⁹-(3-propionsäure)-adenin

### 9a) N⁶-(1-(5-(tert-Butyloxycarbonylamino)butyl))-N⁹-(3-propionsäure-tert-butylester)-adenin

Synthese analog 1 b aus 282 mg (1.0 mmol) N⁹-(3-Propionsäure-tert-butylester)-6-chlorpurin (Beispiel 7a) und 243 mg (1.2 mmol) 5-(tert-Butyloxycarbonylamino)-1-pentylamin. Ausbeute: 219 mg (41 %).
MS (ES+): m/e = 449.3 (100 %; (M+H)⁺)

### 9b) N⁶-(1-(5-Aminopentyl))-N⁹-(3-propionsäure)-adenin

Synthese analog Beispiel 1c aus N⁶-(1-(5-(tert-Butyloxycarbonylamino)pentyl))-N⁹-(3-propionsäure-tert-butylester)-adenin (Beispiel 9a). Ausbeute: 100 %.
¹H-NMR (200 MHz, DMSO): δ = 1.39 (m, 2H, CH₂) 1.50-1.67 (m, 4H, 2 x CH₂); 2.79 (dt, 2H, NH-CH₂); 2.89 (m, 2H, CH₂-C(O)); 3.48 (m, 2H, CH₂-NH₂); 4.37 (t, 2H, N⁹-CH₂); 7.67 (s, breit, 2H, NH₂); 8.04 (t, 1 H, NH); 8.13 + 8.25 (2 s, 2H, C⁶-H + C⁸-H). MS (ES+): m/e = 293.1 (100 %; (M+H)⁺).

### 9c) N⁶-(1-(5-Guanidinopentyl))-N⁹-(3-propionsäure)-adenin

Synthese analog Beispiel 1d aus N⁶-(1-(5-Aminopentyl))-N⁹-(3-propionsäure)-adenin (Beispiel 9b). Ausbeute: 37 %.
¹H-NMR (200 MHz, DMSO): δ = 1.38-1.79 (m, 6H, 3 x CH₂); 2.80 (t, 2H, NH-CH₂); 3.12 (m, 2H, CH₂-C(O)); 3.58 (m, 2H, CH₂-Gua); 4.43 (t, 2H, N⁹-CH₂); 8.07 + 8.21 (2 s, 2H, C⁶-H + C⁸-H).
MS (FAB): m/e = 335.2 (100 %; (M+H)⁺).

### Beispiel 10

### N⁶-(2-Essigsäure)-N⁹-(1-(5-aminopentyl))-adenin

### 10a) N⁶-(2-Essigsäure-tert-butylester)-adenin

155 mg (1 mmol) 6-Chlorpurin und 420 mg (2 mmol) Glycin-tert-butylester Hydrochlorid (80 %ig) wurden in 5 ml absol. DMF gelöst und mit 0.17 ml DIPEA und einer Spatelspitze Kaliumiodid versetzt und 6 h bei 50°C gerührt. Das Lösungsmittel wurde abgedampft und der Rückstand über Kieselgel chromatographiert (Toluol:EE 1:1 bis 1:2). Ausbeute: 76 mg (31 %)
MS (ES+): 250.0 (M+H, 10 %); 193.9 (95), 163.9 (100).

### 10b) N⁶-(2-Essigsäure)-N⁹-(1-(5-(tert-butyloxycarbonylamino)pentyl))-adenin

75 mg (0.3 mmol) N⁶-(2-Essigsäure-tert-butylester)-adenin (Beispiel 10a), 214 mg (0.6 mmol) 4-Toluolsulfonsäure-(5-(tert-butyloxycarbonylamino)pentyl)ester und 42 mg (0.3 mmol) K₂CO₃ wurden in 6 ml absol. DMF gelöst und 5 Tage bei RT gerührt. Das Lösungsmittel wurde abgedampft und der Rückstand über Kieselgel chromatographiert (Toluol:EE 7:3 bis 1:2). Ausbeute: 92 mg (71 %).
MS (ES+): 435.3 (M+H, 25 %); 349.3 (100).

### 10c) N⁶-(2-Essigsäure)-N⁹-(1-(5-aminopentyl))-adenin

Synthese analog Beispiel 1c aus N⁶-(2-Essigsäure)-N⁹-(1-(5-(tert-butyloxycarbonylamino)pentyl))-adenin (Beispiel 10b). Ausbeute: 93 %.
MS (ES+): m/e = 279.2 (15 %; (M+H)⁺, 249.1 (100).

### Beispiel 11

### N⁶-(2-(N-(2-Aminoethyl)-acetamid))-N⁹-(2-essigsäure)-adenin

### 11a) N⁹-(2-Essigsäure-tert-butylester)-adenin

6.76 g (0.05 mol) Adenin wurden in 300 ml absol. DMF unter N₂ suspendiert, danach wurden 2.4 g (0.06 mol) NaH-Dispersion zugegeben und 2 h bei RT gerührt. Innerhalb 30 min wurden 14.7 ml (0.1 mol) Bromessigsäure-tert-butylester zugetropft, wobei sich eine klare Lösung bildete. Es wurde weitere 5 h bei RT gerührt. Das Lösungsmittel wurde abgedampft, der Rückstand mit 500 ml Wasser verrührt, abgesaugt und aus Ethanol kristallisiert. Ausbeute: 5.1 g (41 %).
¹H-NMR (200 MHz, DMSO): δ = 1.42 (s, 9H, tBu); 4.95 (s, 2H, N⁹-CH₂); 7.22 (s, breit, 2H, N⁶H₂); 8.10 + 8.15 (2 s, 2H, C⁶-H + C⁸-H).
MS (ES+): m/e = 250.1 (M+H⁺, 65 %), 194.0 (100).

### 11 b) N⁶-(2-Essigsäure-ethylester)-N⁹-(2-essigsäure-tert-butylester)-adenin

978 mg (3 mmol) NaH und 250 mg (1 mmol) N⁹-(2-Essigsäure-tert-butylester)-adenin (Beispiel 11a) wurden in 10 ml absol. DMF suspendiert und 0.12 ml Chloressigsäureethylester während 10 min zugetropft. Danach wurde 6 h bei 50°C gerührt, dann nochmals die gleiche Menge CsCO₃ zugegeben und 6 h bei 50°C gerührt. Das Lösungsmittel wurde abgedampft und der Rückstand zwischen Wasser und EE verteilt. Die organische Phase wurde getrocknet und eingeengt. Ausbeute: 16%.
¹H-NMR (200 MHz, DMSO): δ = 1.20 (t, 3H, CH₂-CH₃); 1.41 (s, 9H, tBu); 4.00-4.28 (m, 4H, CH₂-CH₃ + N⁶-CH₂); 4.98 (s, 2H, N⁹-CH₂); 8.09 (s, breit, 1H, N⁶H); 8.15 + 8.21 (2 s, 2H, C⁶-H + C⁸-H).
MS (ES+): m/e = 336.3 (M+H⁺, 100 %); 280.3 (60).

### 11c) N⁶-(2-Essigsäure)-N⁹-(2-essigsäure-tert-butylester)-adenin

249 mg (0.74 mmol) N⁶-(2-Essigsäureethylester)-N⁹-(2-essigsäure-tert-butylester)-adenin (Beispiel 11b) wurden in 6 ml Dioxan:Wasser:Triethylamin gelöst und 4 Tage bei RT gerührt. Das Lösungsmittel wurde abgedampft und der Rückstand über Kieselgel chromatographiert (DCM:MeOH 95:5 bis 90:10). Ausbeute: 36 %.
MS (ES+): m/e = 308.3 (M+H⁺, 100 %).

### 11d) N⁶-(2-(N-(2-tert-Butyloxycarbonylaminoethyl)-acetamid))-N⁹-(2-essigsäure-tert-butylester)-adenin

80 mg (0.26 mmol) N⁶-(2-Essigsäure)-N⁹-(2-essigsäure-tert-butylester)-adenin (Beispiel 11c), 42 mg (0.26 mmol) 2-tert-Butyloxycarbonylaminoethylamin wurden unter Argon in 5 ml absol. DMF gelöst und bei 0°C mit 85 mg (0.26 mmol) TOTU und 0.13 ml (0.78 mmol) DIPEA versetzt und 10 min bei 0°C und 2.5 h bei RT gerührt. Es wurde mit EE auf 100 ml verdünnt, dann mit gesättigter Kaliumhydrogencarbonat-Lösung gewaschen, getrocknet und eingeengt. Es wurde über Kieselgel chromatographiert (DCM:MeOH 98:2 bis 90:10). Ausbeute: 5 %.
MS (ES+): m/e = 450.3 (M+H⁺, 100 %).

### 11e) N⁶-(2-(N-(2-Aminoethyl)-acetamid))-N⁹-(2-essigsäure)-adenin

Synthese analog Beispiel 1 c aus N⁶-(2-(N-(2-tert-Butyloxycarbonylaminoethyl)-acetamid))-N⁹-(2-essigsäure-tert-butylester)-adenin (Beispiel 11 d).
Ausbeute: 80 %.
MS (ES+): m/e = 293.1 (100 %; (M+H)⁺).

### Beispiel 12

### N⁶-(4-(2S-(Benzyloxycarbonylamino)buttersäure))-N⁹-(1-(3-guanidinylpropyl))-adenin

### 12a) N⁹-(1-(3-(tert-Butyloxycarbonylamino)propyl))-6-chlorpurin

154.6 mg (1 mmol) 6-Chlorpurin wurden in 2.5 ml absol. DMF gelöst und unter Rühren mit 331.7 mg (2.4 mmol) K₂CO₃ und 285.8 mg (1.2 mmol) N-(3-Brompropyl)carbamidsäure-tert-butylester versetzt. Es wurde 11 h bei RT gerührt, das Lösungsmittel abgedampft, der Rückstand in EE aufgenommen und zweimal mit gesättigter NaHCO₃-Lösung, dann mit NaCl-Lösung gewaschen, getrocknet, filtriert und eingeengt. Der Rückstand wurde über Kieselgel chromatographiert (EE:n-Heptan 8:2). Ausbeute: 267 mg (86 %).
¹H-NMR (200 MHz, DMSO): δ = 1.37 (s, 9H, tBu); 2.00 (tt, 2H, CH₂-CH₂-CH₂); 2.95 (dt, 2H, CH₂-NH); 4.30 (t, 2H, N⁹-CH₂); 6.91 (t, breit, 1H, NH); 8.70 + 8.78 (2 s, 2H, C⁶-H + C⁸-H).
MS (ES+): m/e = 312.2 (100 %; (M+H)⁺); 256.1 (20).

### 12b) N⁶-(4-(2S-(Benzyloxycarbonylamino)buttersäure))-N⁹-(1-(3-(tert-butyloxycarbonylamino)propyl))-adenin

370 mg (1.19 mmol) N⁹-(1-(3-(tert-Butyloxycarbonylamino)propyl))-6-chlorpurin (Beispiel 12a) wurden in 10 ml absol. DMF und 5 ml DIPEA gelöst. Bei RT wurden 449 mg (1.8 mmol) 2S-Benzyloxycarbonylamino-4-aminobuttersäure zugegeben und 50 h bei 65°C gerührt. Das Lösungsmittel wurde abgedampft und der Rückstand zwischen EE und gesättigter NaCl-Lösung ( 20 % KHSO₄) verteilt. Die organische Phase wurde mit Wasser gewaschen, getrocknet, filtriert und eingeengt. Der Rückstand wurde über Kieselgel chromatographiert (EE:MeOH 8:2). Ausbeute: 331 mg (53 %).
¹H-NMR (200 MHz, DMSO): δ = 1.39 (s, 9H, tBu); 1.73-2.21 (m, 2H, CH₂-CH(NH-Z)); 1.90 (m, 2H, CH₂-CH₂-CH₂); 2.92 (dt, 2H, CH₂-NHBoc); 3.15 (dt, 2H, N⁶H-CH₂); 3.88-4.10 (m, 1H, CH-NHZ); 4.14 (t, 2H, N⁹-CH₂); 5.03 (s, 2H, CH₂-Ph); 6.91 (t, breit, 1H, NH-Boc); 7.37 (s, 5H, Ar-H); 7.55-7.81 (m, 2H, NH-Z + N⁶H-CH₂); 8.13 + 8.19 (2 s, 2H, C⁶-H + C⁸-H).
MS (ES+): m/e = 528.2 (100 %; (M+H)⁺).

### 12c) N⁶-(4-(2S-(Benzyloxycarbonylamino)buttersäure))-N⁹-(1-(3-aminopropyl))-adenin

30 mg (0.06 mmol) N⁶-(3-(2S-(Benzyloxycarbonylamino)propionsäure))-N⁹-(1-(3-(tert-butyloxycarbonylamino)propyl))-adenin (Beispiel 12b) wurden in 2 ml 90 %iger Trifluoressigsäure gelöst, 70 min bei RT gerührt, eingeengt und der Rückstand mehrfach mit Ether verrührt. Der Rückstand wurde in Wasser gelöst und gefriergetrocknet. Ausbeute: 100 %.
MS (ES+): m/e = 428.2 (100 %; (M+H)⁺); 294.1 (90).

### 12d) N⁶-(4-(2S-(Benryloxycarbonylamino)buttersäure))-N⁹-(1-(3-guanidinylpropyl))-adenin

Synthese analog Beispiel 1d aus N⁶-(3-(2S-(Benzyloxycarbonylamino)propionsäure))-N⁹-(1-(3-aminopropyl))-adenin
(Beispiel12c). Ausbeute: 77 %.
MS (ES+): m/e = 470.3 (25 %; (M+H)⁺); 336.2 (100).

### Beispiel 13

### N⁶-(4-(2S-(Benzyloxycarbonylamino)buttersäure))-N⁹-(1-(3-(4,5-dihydro-1H-imidazol-2-ylamino)-propyl))-adenin

Synthese analog Beispiel 4 aus N⁶-(3-(2S-(Benzyloxycarbonylamino)propionsäure))-N⁹-(1-(3-aminopropyl))-adenin
(Beispiel12c). Ausbeute: 63 %.
MS (ES+): m/e = 496.3 (100 %; (M+H)⁺).

### Beispiel 14

### N⁶-(3-(2S-(Benzyloxycarbonylamino)propionsäure))-N⁹-(1-(5-guanidinylpentyl))-adenin

### 14a) N⁹-(1-(5-(tert-Butyloxycarbonylamino)pentyl))-6-chlorpurin

Synthese analog Beispiel 12a aus 6-Chlorpurin und N-(5-Tosyloxypentyl)carbamidsäure-tert-butylester. Ausbeute: 66 %.
¹H-NMR (200 MHz, DMSO): δ = 1.11-1.48 (m, 4H, 2 x CH₂); 1.35 (s, 9H, tBu); 1.87 (tt, 2H, CH₂); 2.97 (dt, 2H, CH₂-NHBoc); 4.28 (t, 2H, N⁹-CH₂); 6.72 (t, breit, 1H, NH); 8.71 + 8.78 (2 s, 2H, C⁶-H + C⁸-H).
MS (ES+): m/e = 340.2 (100 %; (M+H)⁺); 284.1 (50).

### 14b) N⁶-(3-(2S-(Benzyloxycarbonylamino)propionsäure))-N⁹-(1-(5-(tert-butyloxycarbonylamino)pentyl))-adenin

Synthese analog Beispiel 12b aus N⁹-(1-(5-(tert-Butyloxycarbonylamino)pentyl))-6-chlorpurin und 2S-Benzyloxycarbonylamino-3-aminopropionsäure. Ausbeute: 23 %. ¹H-NMR (200 MHz, DMSO): δ = 1.10-1.49 (m, 4H, 2 x CH₂; 1.36 (s, 9H, tBu); 1.62-1.88 (m, 2H, CH₂); 2.87 (dt(2H, CH₂-NHBoc); 3.68-4.98 (m, 5H, N⁹-CH₂ + CH₂-CH-NHZ); 5.00 (s, 2H, CH₂-Ph); 6.75 (t, breit, 1H, NH); 8.02 + 8.20 (2 s, 2H, C⁶-H + C⁸-H).
MS (FAB): m/e = 542.3 (100 %; (M+H)⁺).

### 14c) N⁶-(3-(2S-(Benzyloxycarbonylamino)propionsäure))-N⁹-(1-(5-aminopentyl))-adenin

Synthese analog Beispiel 12c aus N⁶-(3-(2S-(Benzyloxycarbonylamino)propionsäure))-N⁹-(1-(5-(tert-butyloxycarbonylamino)pentyl))-adenin (Beispiel 14b). Ausbeute: 100 %.
¹H-NMR (200 MHz, DMSO): δ = 1.18-1.40 + 1.44-1.65 + 1.71-1.93 (2 m, 6H, 3 x CH₂); 2.77 (dt(2H, CH₂-NHBoc); 3.64-4.35 (m, 5H, N⁹-CH₂ + CH₂CH-NHZ); 5.00 (s, 2H, CH₂-Ph); 7.66 (m, 3H, NH₃⁺); 8.20 + 8.24 (2 s, 2H, C⁶-H + C⁸-H). MS (ES+): m/e = 442.3 (40 %; (M+H)⁺); 308.2 (100).

### 14d) N⁶-(3-(2S-(Benzyloxycarbonylamino)propionsäure))-N⁹-(1-(5-guanidinylpentyl))-adenin

Synthese analog Beispiel 1d aus N⁶-(3-(2S-(Benzyloxycarbonylamino)propionsäure))-N⁹-(1-(5-aminopentyl))-adenin (Beispiel 14c). Ausbeute: 90 %.
MS (ES+): m/e = 484.3 (70 %; (M+H)⁺); 350.2 (60).

### Beispiel 15

### N⁶-(3-(2S-(Benzyloxycarbonylamino)propionsäure))-N⁹-(1-(5-(4,5-dihydro-1H-imidazol-2-ylamino)pentyl))-adenin

Synthese analog Beispiel 4 aus N⁶-(3-(2S-(Benryloxycarbonylamino)propionsäure)-N⁹-(1-(5-aminopentyl))-adenin (Beispiel 14c). Ausbeute: 75 %.
MS (ES+): m/e = 510.3 (40 %; (M+H)⁺); 376.2 (100).

### Beispiel 16

### N⁶-(3-(2S-(Benzyloxycarbonylamino)propionsäure))-N⁹-(1-(3-guanidinylpropyl))-adenin

### 16a) N⁶-(3-(2S-(Benzyloxycarbonylamino)propionsäure))-N⁹-(1-(3-(tert-butyloxycarbonylamino)propyl))-adenin

Synthese analog Beispiel 12b aus N⁹-(1-(3-(tert-Butyloxycarbonylamino)propyl))-6-chlorpurin (Beispiel 12a) und 2S-Benzyloxycarbonylamino-3-aminopropionsäure. Ausbeute: 27 %.
¹H-NMR (200 MHz, DMSO): δ = 1.37 (s, 9H, tBu); 1.90 (m, 2H, CH₂-CH₂-CH₂); 2.92 (dt, 2H, CH₂-NHBoc); 3.86 (m, breit, 2H, CH₂-CH(NH-Z)); 4.13 (t, 2H, N⁹-CH₂); 4.40 (m, 1H, CH-NHZ); 5.01 (s, 2H, CH₂-Ph); 6.92 (t, breit, 1H, NH-Boc); 7.33 (s, 5H, Ar-H); 7.55-7.75 (m, 2H, NH-Z + N⁶H-CH₂); 8.16 + 8.22 (2 s, 2H, C⁶-H + C⁸-H).
MS (ES+): m/e = 514.3 (100 %; (M+H)⁺).

### 16b) N⁶-(3-(2S-(Benzyloxycarbonylamino)propionsäure))-N⁹-(1-(3-aminopropyl))-adenin

Synthese analog Beispiel 12c aus N⁶-(3-(2S-(Benzyloxycarbonylamino)-propionsäure))-N⁹-(1-(3-(tert-butyloxycarbonylamino)propyl))-adenin (Beispiel 16a). Ausbeute: 100%.
MS (ES+): m/e = 414.2 (100 %; (M+H)⁺); 280.2 (70).

### 16c) N⁶-(3-(2S-(Benzyloxycarbonylamino)propionsäure))-N⁹-(1-(3-guanidinylpropyl))-adenin

Synthese analog Beispiel 1d aus N⁶-(3-(2S-(Benzyloxycarbonylamino)propionsäure))-N⁹-(1-(3-aminopropyl))-adenin (Beispiel 16b). Ausbeute: 98 %.
MS (ES+): m/e = 456.3 (40 %; (M+H)⁺); 322.2 (100).

### Beispiel 17

### N⁶-(3-(2S-(Benzyloxycarbonylamino)propionsäure))-N⁹-(1-(4-guanidinylbutyl))-adenin

### 17a) N⁹-(1-(4-tert-Butyloxycarbonylamino)butyl))-6-chlorpurin

Synthese analog Beispiel 12a aus 6-Chlorpurin und N-(4-Tosyloxybutyl)carbamidsäure-tert-butylester. Ausbeute: 66 %.
¹H-NMR (200 MHz, DMSO): δ = 1.30 (m, 2H, CH₂); 1.35 (s, 9H, tBu); 1.86 (tt, 2H, CH₂); 2.93 (dt, 2H, CH₂-NHBoc); 4.31 (t, 2H, N²-CH₂); 6.79 (t, breit, 1H, NH); 8.72 + 8.78 (2 s, 2H, C⁶-H + C⁸-H).
MS (ES+): m/e = 326.2 (80 %; (M+H)⁺); 270.1 (100).

### 17b) N⁶-(3-(2S-(Benzyloxycarbonylamino)propionsäure))-N⁹-(1-(4-(tert-butyloxycarbonylamino)butyl))-adenin

Synthese analog Beispiel 12b aus N⁹-(1-(4-tert-Butyloxycarbonylamino)butyl))-6-chlorpurin (Beispiel 17a) und 2S-Benzyloxycarbonylamino-3-aminopropionsäure.
Ausbeute: 33%.
¹H-NMR (200 MHz, DMSO): δ = 1.30 (m, 2H, CH₂); 1.35 (s, 9H, tBu); 1.75 (m, 2H, CH₂); 2.91 (dt(2H, CH₂-NHBoc); 3.71-4.34 (m,5H, CH₂-CH(NH-Z) + N⁹-CH₂); 5.01 (s, 2H, CH₂-Ph); 6.89 (t, breit, 1H, NH-Boc); 7.35 (s, 5H, Ar-H); 7.46-7.73 (m, 2H, NH-Z + N⁶H-CH₂); 8.10 (breit) + 8.20 (2 s, 2H, C⁶-H + C⁸-H).
MS (FAB): m/e = 528.4 (100 %; (M+H)⁺).

### 17c) N⁶-(3-(2S-(Benzyloxycarbonylamino)propionsäure))-N⁹-(1-(4-aminobutyl))-adenin

Synthese analog Beispiel 12c aus N⁶-(3-(2S-(Benzyloxycarbonylamino)propionsäure))-N⁹-(1-(4-tert-butyloxycarbonylaminobutyl))-adenin (Beispiel 17b). Ausbeute: 100 %.
¹H-NMR (200 MHz, DMSO): δ = 1.48 (m, 2H, CH₂); 1.87 (m, 2H, CH₂); 2.80 (dt, 2H, CH₂-NH₂); 3.69-4.02 (m, 2H, CH₂-CH(NH-Z)); 4.20 (t, 2H, N⁹-CH₂); 4.36 (m,1H, CH(NH-Z)); 5.01 (s, 2H, CH₂-Ph); 7.33 (s, 5H, Ar-H); 7.64 (s, breit, 4H, NH₃⁺ + N⁶H-CH₂); 8.10 (breit) + 8.20 (2 s, 2H, C⁶-H + C⁸-H).
MS (ES+): m/e = 428.3 (50 %; (M+H)⁺); 294.2 (100).

### 17d) N⁶-(3-(2S-(Benzyloxycarbonylamino)propionsäure))-N⁹-(1-(4-guanidinylbutyl))-adenin

Synthese analog Beispiel 1d aus N⁶-(3-(2S-(Benzyloxycarbonylamino)propionsäure))-N⁹-(1-(4-aminobutyl))-adenin (Beispiel 17c). Ausbeute: 78 %.
MS (ES+): m/e = 470.2 (50 %; (M+H)⁺); 336.2 (100).

### Beispiel 18

### N⁶-(3-(2S-(Benzyloxycarbonylamino)propionsäure))-N⁹-(1-(4-(4,5-dihydro-1H-imidazol-2-yl)amino)butyl))-adenin

Synthese analog Beispiel 4 aus N⁶-(3-(2S-(Benzyloxycarbonylamino)propionsäure))-N⁹-(1-(4-aminobutyl))-adenin (Beispiel17c). Ausbeute: 41%.
MS (ES+): m/e = 496.3 (60 %; (M+H)⁺); 362.2 (100).

### Beispiel 19

### 2S-Benzyloxycarbonylamino-3-(6-(4-(1,4,5,6-tetrahydropyrimidin-2-ylcarbamoyl)-piperdin-1-yl)-purin-9-yl)-propionsäure

### 19a) 2S-Benzyloxycarbonylamino-3-(6-(4-carboxy-piperidin-1-yl)-purin-9-yl)-propionsäure-tert-butylester

260 mg (0.6 mmol) 2S-Berizyloxycarbonylamino-3-(6-chlor-purin-9-yl)-propionsäure-tert-butylester (Beispiel 1a), 116.3 mg (0.9 mmol) Piperidin-4-carbonsäure und 310 mg (2.4 mmol) DIPEA in 4 ml absol. DMF wurden 16 h bei 60°C gerührt. Danach wurden weitere 310 mg DIPEA zugegeben und nochmals 24 h bei 60°C gerührt. Das Lösungsmittel wurde abgedampft und der Rückstand zwischen EE und Wasser verteilt. Die organische Phase wurde nochmals mit KHSO₄/K₂SO₄-Lösung, dann mit NaCl-Lösung gewaschen, getrocknet, filtriert und eingeengt. Der Rückstand wurde über Kieselgel chromatographiert (EE). Ausbeute: 219 mg (69 %).
MS (ES+): m/e = 525.3 (100 %; (M+H)⁺).

### 19b) 2S-Benzyloxycarbonylamino-3-(6-(4-(1,4,5,6-tetrahydropyrimidin-2-ylcarbamoyl)-piperidin-1-yl)-purin-9-yl)-propionsäure-tert-butylester

126 mg (0.24 mmol) 2S-Benzyloxycarbonylamino-3-(6-(4-carboxy-piperidin-1-yl)-purin-9-yl)-propionsäure-tert-butylester (Beispiel 19a), 39.3 mg (0.29 mmol) 2-Amino-1,4,5,6-tetrahydropyrimidin-Hydrochlorid, 86.6 mg (0.264 mmol) TOTU (O-((ethoxycarbonyl)cyanomethylenamino)-N,N,N',N'-tetramethyluronium-tetrafluoroborat (W. König et al., Proceedings of the 21 st European Peptide Symposium 1990, E. Giralt, D. Andreu, Eds., ESCOM, Leiden, S. 143) und 124 mg DIPEA wurden nacheinander zu 3 ml absol. DMF zugegeben. Es wurde 3 h bei RT gerührt, danach wurden weitere 28 mg DIPEA zugegeben und 12 h bei RT gerührt.

Das Reaktionsgemisch wurde mit Eisessig/Toluol (1:1) auf pH 6 eingestellt, die Reaktionslösung eingeengt, der Rückstand zwischen EE und gesättigter NaHCO₃-Lösung verteilt, die organische Phase mit NaCl gewaschen, getrocknet und eingeengt. Der Rückstand wurde über Kieselgel chromatographiert (EE:MeOH:TEA 85:15:1.5). Ausbeute: 70 mg.
MS (ES+): m/e = 606.4 (60 %; (M+H)⁺); 416.3 (40); 275.7 (100).

### 19c) 2S-Benzyloxycarbonylamino-3-(6-(4-(1,4,5,6-tetrahydropyrimidin-2-ylcarbamoyl)-piperidin-1-yl)-purin-9-yl)-propionsäure

80 mg 2S-Benzyloxycarbonylamino-3-(6-(4-(1,4,5,6-tetrahydropyrimidin-2-ylcarbamoyl)-piperidin-1-yl)-purin-9-yl)-propionsäure-tert-butylester (Beispiel 19b) wurden in 16 ml vorgekühlter 95 %iger Trifluoressigsäure gelöst und zuerst 30 min bei 0°C, dann 30 min bei RT gerührt. Die Trifluoressigsäure wurde abrotiert, der Rückstand dreimal mit Toluol koevaporiert, in Ethanol/Ether (1:2) verrührt, mit Ether gewaschen und im Vakuum getrocknet. Ausbeute: 59 mg.
MS (ES+): m/e = 550.3 (60 %; (M+H)⁺); 416.3 (100).

### Beispiel 20

### 2S-Benzyloxycarbonylamino-3-(1-(9-(2-guanidinoethyl)-9H-purin-6-yl)-1H-imidazol-4-yl)-propionsäure

### 20a) N⁹-(1-(2-(tert-Butyloxycarbonylamino)ethyl))-6-chlorpurin

Die Synthese erfolgte analog Beispiel 12a aus 6-Chlorpurin und N-(2-Tosyloxyethyl)carbamidsäure-tert-butylester. Ausbeute: 36 %.
¹H-NMR (200 MHz, DMSO): δ = 1.24 (s, 9H, tBu); 3.40 (dt, 2H, CH₂-NHBoc); 4.35 (t, 2H, N⁹-CH₂); 6.91 (t, breit, 1H, NH); 8.60 + 8.78 (2 s, 2H, C6-H + C8-H). MS (FAB): m/e = 298.2 (100 %; (M+H)⁺).

### 20b) 2S-Benzyloxycarbonylamino-3-(1-(9-(2-(tert-butyloxycarbonylamino)ethyl)-9H-purin-6-yl)-1H-imidazol-4-yl)-propionsäure

Die Synthese erfolgte analog Beispiel 12b aus N⁹-(1-(2-(tert-Butyloxycarbonylamino)ethyl))-6-chlorpurin (Beispiel 21a) und N_{α}-Z-L-Histidin. Ausbeute: 33 %.
MS (ES+): m/e = 551.3 (100 %; (M+H)⁺).

### 20c) 3-(1-(9-(2-Aminoethyl)-9H-purin-6-yl)-1H-imidazol-4-yl)-2S-benzyloxycarbonylamino-propionsäure

Die Synthese erfolgte analog Beispiel 12c aus 2S-Benzyloxycarbonylamino-3-(1-(9-(2-(tert-butyloxycarbonylamino)ethyl)-9H-purin-6-yl)-1H-imidazol-4-yl)-propionsäure (Beispiel 20b). Ausbeute: 100 %.
MS (ES+): m/e = 451.3 (70 %; (M+H)⁺);
¹H-NMR (200 MHz, DMSO): δ = 2.87-3.15 (m, 2H, Im-CH₂); 3.38-3.51 (m, 2H, CH₂-NH₂); 4.36 (m,1H, CH-NHZ); 4.60 (t, 2H, N⁹-CH₂); 5.00 (s, 2H, CH₂-Ph); 7.28 (s, 5H, Aryl-H); 7.62 (d, 1H, NH-Z); 8.23 + 9.05 (2 s, 2H, Im-H); 8.71 + 8.88 (2 s, 2H, C6-H + C8-H).

### 20d) 2S-Benzyloxycarbonylamino-3-(1-(9-(2-guanidinoethyl)-9H-purin-6-yl)-1H-imidazol-4-yl)-propionsäure

Die Synthese erfolgte analog Beispiel 1d aus 3-(1-(9-(2-Aminoethyl)-9H-purin-6-yl)-1 H-imidazol-4-yl)-2S-benzyloxycarbonylamino-propionsäure (Beispiel 20c): Ausbeute: 38 %.
MS (ES+): m/e = 493.3 ((M+H)⁺);

### Beispiel 21

### 2R-Benzyloxycarbonylamino-3-(6-N-(4-guanidinocyclohexyl)amino)-purin-9-yl)-propionsäure

### 21a) N⁹-(3-(2R-(Benzyloxycarbonylamino)-tert-butylpropionat))-6-chlorpurin

Die Synthese erfolgte analog Beispiel 1a aus 6-Chlorpurin und N-Benzyloxycarbonyl-D-serin-tert-butylester.
MS (FAB): m/e = 432.2 (100 %; (M+H)⁺); 376.1 (30).

### 21 b) 2R-Benzyloxycarbonylamino-3-(6-(N-(4-(tert-butyloxycarbonylamino)cyclohexyl)amino)-purin-9-yl)-propionsäure-tert-butylester

Die Synthese erfolgte analog Beispiel 1 b aus 4-Amino-1-(tert-butyloxycarbonylamino)-cylohexan und N⁹-(3-(2R-(Benzyloxycarbonylamino)-tert-butylpropionat))-6-chlorpurin (Beispiel 21a). Ausbeute: 55 %.
MS (FAB): m/e = 610.3 (100 %; (M+H)⁺).

### 21c) 3-(6-(N-(4-Aminocyclohexyl)amino)-purin-9-yl)-2R-benzyloxycarbonylamino-propionsäure

Die Synthese erfolgte analog Beispiel 1 c aus 2R-Benzyloxycarbonylamino-3-(6-(N-(4-(tert-butyloxycarbonylamino)cyclohexyl)amino)-purin-9-yl)-propionsäure-tert-butylester (Beispiel 21 b). Ausbeute: 100 %.
MS (ES+): m/e = 454.2 (50%, (M+H)⁺).

### 21 d) 2R-Benzyloxycarbonylamino-3-(6-(N-(4-guanidinocyclohexyl)amino)-purin-9-yl)-propionsäure

Die Synthese erfolgte analog Beispiel 1 d aus 3-(6-(N-(4-Aminocyclohexyl)amino)-purin-9-yl)-2R-benzyloxycarbonylamino-propionsäure (Beispiel 21c). Ausbeute: 80 %.
MS (ES+): m/e = 496.3 (50 %, (M+H)⁺).

### Beispiel 22

### 2R-Benzyloxycarbonylamino-3-(6-(N-3-guanidinomethylbenzyl)amino)-purin-9-yl)-propionsäure

### 22a) 2R-Benzyloxycarbonylamino-3-(6-(N-(3-tert-butyloxycarbonylaminomethylbenzyl)amino)-purin-9-yl)-propionsäure-tert-butylester

Die Synthese erfolgte analog Beispiel 1 b aus 3-Aminomethyl-1-(tert-butyloxycarbonylaminomethyl)-benzol und N⁹-(3-(2R-(Benzyloxycarbonylamino)-tert-butylpropionat)-6-chlorpurin (Beispiel 21 a). Ausbeute: 51 %.
MS (ES+): m/e = 632.3 (100 %; (M+H)⁺).

### 22b) 3-(6-(N-(3-Aminomethylbenzyl)amino)-purin-9-yl)-2R-benzyloxycarbonylamino-propionsäure

Die Synthese erfolgte analog Beispiel 1c aus 2R-Benzyloxycarbonylamino-3-(6-(N-(3-tert-butyloxycarbonylaminomethylbenzyl)amino)-purin-9-yl)-propionsäure-tert-butylester (Beispiel 22a). Ausbeute: 100 %.
MS (ES+): m/e = 476.2 ((M+H)⁺, 50 %); 342.2 (70).

### 22c) 2R-Benzyloxycarbonylamino-3-(6-(N-(3-guanidinomethylbenzyl)amino)-purin-9-yl)-propionsäure

Die Synthese erfolgte analog Beispiel 1 d aus 3-(6-(N-(3-Aminomethylbenzyl)amino)-purin-9-yl)-2R-benzyloxycarbonylamino-propionsäure (Beispiel 22b). Ausbeute: 30 %.
MS (ES+): m/e = 518.3 ((M+H)+, 20 %).

### Beispiel 23

### 3-(6-((4-(Benzimidazol-2-ylamino)-butyl)-amino)-purin-9-yl)-2S-benzyloxycarbonyl-amino-propionsäure

### 23a) 1-(4-tert-Butyloxycarbonylamino-butyl)-3-(2-nitro-phenyl)-thiohamstoff

Zu 0.97 g (5.15 mmol) 4-(tert-Butyloxycarbonylaminobutyl)-1-amin in 25 ml absol. DMF wurden bei 0°C 0.928 g (5.15 mmol) 2-Nitrophenylisothiocyanat in 5 ml absol. DMF zugetropft. Danach wurde 1 h bei 0°C gerührt. Das Lösungsmittel wurde abdestilliert und der Rückstand über Kieselgel chromatographiert (EE:n-Heptan 1:2 bis 1:1). Ausbeute: 1.8 g (95 %).
MS (ES+): m/e = 369.2 (M+H)⁺, 100 %).

### 23b) 3-(2-Amino-phenyl)-1-(4-tert-butyloxycarbonylamino-butyl)-thioharnstoff

1.78 g (4.8 mmol) 1-(4-tert-Butyloxycarbonylamino-butyl)-3-(2-nitro-phenyl)-thioharnstoff (Beispiel 23a) wurden in 120 ml Methanol gelöst und bei RT 3 h über 1 g Pd/C hydriert (1 bar). Der Katalysator wurde abfiltriert, das Filtrat eingeengt und über Kieselgel chromatographiert (EE:n-Heptan 1:1). Ausbeute: 1.4 g.

### 23c) 4-(Benzimidazol-2-ylamino)-1-(tert-butyloxycarbonylamino)-butan

Zu 1.4 g (4.14 mmol) 3-(2-Amino-phenyl)-1-(4-tert-butyloxycarbonylamino-butyl)-thioharnstoff (Beispiel 23b) in 30 ml Ethanol wurden 1.79 g (8.28 mmol) gelbes Quecksilberoxid und 27 mg Schwefelblume zugegeben und das Reaktionsgemisch für 3 h auf 50-55°C erhitzt. Es wurde abgesaugt und mit Ethanol gewaschen. Das Filtrat wurde eingeengt und das Produkt über Kieselgel chromatographiert (DCM:Methanol 9:5, dann 9:1). Ausbeute: 43 %.
MS (ES+): m/e = 305.2 ( (M+H)⁺, 100 %).

### 23d) 4-(Benzimidazol-2-ylamino)-1-amino-butan

198 mg (0.65 mmol) 4-(Benzimidazol-2-ylamino)-1-(tert-butyloxycarbonylamino)-butan (Beispiel 23c) wurden bei 0°C in 20 ml 95 %iger Trifluoressigsäure gelöst und 2 h bei 0°C gerührt, danach bei RT während 30 min eingeengt. Der Rückstand wurde dreimal mit Toluol koevaporiert, danach mit Ether verrührt und mit Pentan gewaschen und im Vakuum getrocknet. Ausbeute: 100 %.
MS (ES+): m/e = 205.2 ((M+H)⁺, 100 %).

### 23e) 3-(6-((4-(Benzimidazol-2-ylamino)-butyl)-amino)-purin-9-yl)-2S-benzyloxycarbonylamino-propionsäure-tert-butylester

Die Synthese erfolgte analog Beispiel 1 b aus 4-(Benzimidazol-2-ylamino)-1-amino-butan (Beispiel 23d) und N⁹-(3-(2S-(benzyloxycarbonylamino)-tert-butylpropionat))-6-chlorpurin (Beispiel 1a). Ausbeute: 32 %.
MS (ES+): m/e = 600.3 (100 %; (M+H)⁺).

### 23f) 3-(6-((4-(Benzimidazol-2-ylamino)-butyl)-amino)-purin-9-yl)-2S-benzyloxycarbonylamino-propionsäure

Die Synthese erfolgte analog Beispiel 1 c aus 3-(6-((4-(Benzimidazol-2-ylamino)-butyl)-amino)-purin-9-yl)-2S-benzyloxycarbonylamino-propionsäure-tert-butylester (Beispiel 23e). Ausbeute: 100 %.
MS (ES+): m/e = 544.2 ((M+H)⁺, 70 %).

### Beispiel 24

### 2S-Benzyloxycarbonylamino-3-(6-(4-((4,5-dihydro-1H-imidazol-2-ylamino)-methyl)-piperidin-1-yl)-purin-9-yl)-propionsäure

### 24a) 3-(6-(4-(Aminomethyl)-piperidin-1-yl)-purin-9-yl)-2S-benzyloxycarbonylamino-propionsäure-tert-butylester

Die Synthese erfolgte analog Beispiel 1b aus 4-(Aminomethyl)-piperidin und N⁹-(3-(2S-(benzyloxycarbonylamino)-tert-butylpropionat))-6-chlorpurin (Beispiel 1a).
Ausbeute: 96.4 %.
MS (ES+): m/e = 510.3 (100 %; (M+H)⁺).

### 24b) 3-(6-(4-(Aminomethyl)-piperidin-1-yl)-purin-9-yl)-2S-benzyloxycarbonylamino-propionsäure

Die Synthese erfolgte analog Beispiel 1c aus 3-(6-(4-(Aminomethyl)-piperidin-1-yl)-purin-9-yl)-2S-benzyloxycarbonylamino-propionsäure-tert-butylester (Beispiel 24a).
Ausbeute: 100 %.
MS (ES+): m/e = 454.3 ((M+H)⁺, 30 %).

### 24c) 2S-Benzyloxycarbonylamino-3-(6-(4-((4,5-dihydro-1H-imidazol-2-ylamino)-methyl)-piperidin-1-yl)-purin-9-yl)-propionsäure

Die Synthese erfolgte analog Beispiel 4 aus 3-(6-(4-(Aminomethyl)-piperidin-1-yl)-purin-9-yl)-2S-benzyloxycarbonylamino-propionsäure (Beispiel 24b). Ausbeute: 95 %.
MS (ES+): m/e = 522.3 ((M+H)⁺, 40 %).

### Beispiel 25

### 2R-Benzyloxycarbonylamino-3-(6-4-((4,5-dihydro-1H-imidazol-2-ylamino)-methyl)-piperidin-1-yl)-purin-9-yl)-propionsäure

Die Synthese erfolgte analog Beispiel 24 ausgehend von N⁹-(3-(2R-(Benzyloxycarbonylamino)-tert-butylpropionat))-6-chlorpurin (Beispiel 21 a).
MS (ES+): m/e = 522.3 ((M+H)⁺, 20 %).

### Beispiel 26

### 2S-Benzyloxycarbonylamino-3-(6-(4-(guanidinomethyl)-piperidin-1-yl)-purin-9-yl)-propionsäure

Die Synthese erfolgte analog Beispiel 1d aus 3-(6-(4-(Aminomethyl)-piperidin-1-yl)-purin-9-yl)-2S-benzyloxycarbonylamino-propionsäure (Beispiel 24b). Ausbeute: 74 %.
MS (ES+): m/e = 496.3 ((M+H)⁺, 40%).

### Beispiel 27

### 2S-Benzyloxycarbonylamino-3-(6-(3-(3-benzylureido)-phenylsulfanyl)-purin-9-yl)-propionsäure

### 27a) 3-(6-(3-Amino-phenylsulfanyl)-purin-9-yl)-2S-benzyloxycarbonylamino-propionsäure-tert-butylester

0.602 mmol 3-Mercaptoanilin wurden zusammen mit 0.602 mmol N⁹-(3-(2S-(Benzyloxycarbonylamino)-tert-butyl-propionat))-6-chlorpurin (Beispiel 1a) in DMF und DIPEA während 12 h gerührt. Die Reaktionslösung wurde eingeengt, der Rückstand zwischen EE und gesättigter NaHCO₃-Lösung verteilt, die Phasen wurden getrennt, die organische Phase wurde mit halbgesättigter NaHCO₃-Lösung und NaCl-Lösung gewaschen, getrocknet, eingeengt und das Produkt über Kieselgel chromatographiert (EE:Heptan 1:1). Ausbeute: 190 mg.
MS (ES+): m/e = 521.3 ((M+H)⁺, 100 %).

### 27b) 2S-Benzyloxycarbonylamino-3-(6-(3-(3-benzylureido)-phenylsulfanyl)-purin-9-yl)-propionsäure-tert-butylester

Zu 180 mg 3-(6-(3-Amino-phenylsulfanyl)-purin-9-yl)-2S-benzyloxycarbonylamino-propionsäure-tert-butylester (Beispiel 27a) in 3 ml absol. Acetonitril wurden 46.1 mg Benzylisocyanat in 1 ml Acetonitril mittels einer Spritze zugegeben. Das Gemisch wurde 48 h bei RT gerührt, eingeengt und der Rückstand über Kieselgel chromatographiert (DCM:EE 7:3 bis 1:1). Ausbeute: 205 mg.
MS (ES+): m/e = 654.4 ((M+H)⁺, 100 %).

### 27c) 2S-Benzyloxycarbonylamino-3-(6-(3-(3-benzylureido)-phenylsulfanyl)-purin-9-yl)-propionsäure

Die Synthese erfolgte analog Beispiel 1 c aus 2S-Benzyloxycarbonylamino-3-(6-(3-(3-benzylureido)-phenylsulfanyl)-purin-9-yl)-propionsäure-tert-butylester (Beispiel 27b). Ausbeute: 100 %.
MS (ES+): m/e = 598.4 ((M+H)⁺, 100 %).

### Beispiel 28

### 2S-Neopentyloxycarbonylamino-3-(6-(4-(1,4,5,6-tetrahydropyrimidin-2-ylcarbamoyl)-piperidin-1-yl)-purin-9-yl)-propionsäure

### 28a) 2S-Amino-3-(6-(4-carboxy-piperidin-1-yl)-purin-9-yl)-propionsäure-tert-butylester

1.7 g 2S-Benzyloxycarbonylamino-3-(6-(4-carboxy-piperidin-1-yl)-purin-9-yl)-propionsäure-tert-butylester (Beispiel 19a) wurden in 200 ml AcOH gelöst und über Pd/C bei 1 atm H₂-Druck hydriert. Der Katalysator wurde abfiltriert, das Lösungsmittel abdestilliert und der Rückstand lyophilisiert. Ausbeute: 100 %.
MS (ES+): m/e = 391.3 ((M+H)⁺, 100 %).

### 28b) 2S-Neopentyloxycarbonylamino-3-(6-(4-carboxy-piperidin-1-yl)-purin-9-yl)-propionsäure-tert-butylester

390 mg (1 mmol) 2S-Amino-3-(6-(4-carboxy-piperidin-1-yl)-purin-9-yl)-propionsäure-tert-butylester (Beispiel 20a) in 4 ml DMF wurden bei 0°C mit 230 mg (1 mmol) N-(Neopentyloxycarbonyloxy)-succinimid und 0.17 ml DIPEA versetzt und nach langsamem Erwärmen 12 h bei RT gerührt. Das Reaktionsgemisch wurde eingeengt und der Rückstand chromatographiert (Lobar-C, DCM:MeOH:AcOH:H₂O 90:10:1:1). Ausbeute: 540 mg.
MS (ES+): m/e = 505.4 ((M+H)⁺, 100 %).

### 28c) 2S-Neopentyloxycarbonylamino-3-(6-(4-(1,4,5,6-tetrahydropyrimidin-2-ylcarbamoyl)-piperidin-1-yl)-purin-9-yl)-propionsäure-tert-butylester

505 mg (1 mmol) 2S-Neopentyloxycarbonylamino-3-(6-(4-carboxy-piparidin-1-yl)-purin-9-yl)-propionsäure-tert-butylester (Beispiel 20b) wurden in 10 ml Acetonitril gelöst, mit 250 mg DCCI und 184 mg Pentafluorphenol versetzt und danach 30 Minuten bei RT gerührt. Es wurde filtriert, die Mutterlauge eingeengt, in 5 ml DMF aufgenommen, mit 200 mg 2-Amino-1,4,5,6-tetrahydropyrimidin versetzt und 12 h bei RT gerührt. Das Lösungsmittel wurde im Vakuum abdestilliert und der Rückstand chromatographiert (Lobar-C, DCM:MeOH:AcOH:H₂O 98:8:0.8:0.8). Ausbeute: 270 mg.
MS (ES+): m/e = 586.5 ((M+H)⁺, 100 %).

### 28d) 2S-Neopentyloxycarbonylamino-3-(6-(4-(1,4,5,6-tetrahydropyrimidin-2-ylcarbamoyl)-piperidin-1-yl)-purin-9-yl)-propionsäure

Die Synthese erfolgte analog Beispiel 19c aus 2S-Neopentyloxycarbonylamino-3-(6-(4-(1,4,5,6-tetrahydropyrimidin-2-ylcarbamoyl)-piperidin-1-yl)-purin-9-yl)-propionsäure-tert-butylester (Beispiel 28c). Ausbeute: 94 %.
MS (ES+): m/e = 530.4 ((M+H)⁺, 20 %).

### Beispiel 29

### 2S-(1-Adamantylmethyloxycarbonylamino)-3-(6-(4-(1,4,5,6-tetrahydropyrimidin-2-ylcarbamoyl)-piperidin-1-yl)-purin-9-yl)-propionsäure

### 29a) 2S-(1-Adamantylmethyloxycarbonylamino)-3-(6-(4-carboxy-piperidin-1-yl)-purin-9-yl)-propionsäure-tert-butylester

Die Synthese erfolgte analog Beispiel 28b aus N-(1-Adamantylmethyloxycarbonyloxy)-succinimid und 2S-Amino-3-(6-(4-carboxy-piperidin-1-yl)-purin-9-yl)-propionsäure-tert-butylester (Beispiel 28a). Ausbeute: 85 %.
MS (ES+): m/e = 583.4 ((M+H)⁺, 100 %).

### 29b) 2S-(1-Adamantylmethyloxycarbonylamino)-3-(6-(4-(1,4,5,6-tetrahydropyrimidin-2-ylcarbamoyl)-piperidin-1-yl)-purin-9-yl)-propionsäure-tert-butylester

Die Synthese erfolgte analog Beispiel 28c aus 2S-(1-Adamantylmethyloxycarbonylamino)-3-(6-(4-carboxy-piperidin-1-yl)-purin-9-yl)-propionsäure-tert-butylester (Beispiel 29a). Ausbeute: 75 %.
MS (ES+): m/e = 664.5 ((M+H)⁺, 30 %).

### 29c) 2S-(1-Adamantylmethyloxycarbonylamino)-3-(6-(4-(1,4,5,6-tetrahydropyrimidin-2-ylcarbamoyl)-piperidin-1-yl)-purin-9-yl)-propionsäure

Die Synthese erfolgte analog Beispiel 19c aus 2S-(1-Adamantylmethyloxycarbonylamino)-3-(6-(4-(1,4,5,6-tetrahydropyrimidin-2-ylcarbamoyl)-piperidin-1-yl)-purin-9-yl)-propionsäure-tert-butylester (Beispiel 29b).
Ausbeute: 100 %.
MS (ES+): m/e = 608.4 ((M+H)⁺, 10 %).

### Pharmakologische Untersuchung

Als Testmethode, nach der beispielsweise die antagonistische Wirkung der erfindungsgemäßen Verbindungen auf den Vitronectinrezeptor αᵥβ₃ bestimmt werden kann, wird nachfolgend die Inhibierung der Bindung von Kistrin an humanen Vitronectinrezeptor (VnR) beschrieben (αᵥβ₃-ELISA-Test; die Testmethode wird bei der Auflistung der Testergebnisse mit "K/VnR" abgekürzt).

### Reinigung von Kistrin

Kistrin wird nach den Methoden von Dennis et al., wie in Proc. Natl. Acad. Sci. USA 1989, 87, 2471-2475 und PROTEINS: Structure, Function and Genetics 1993, 15, 312-321, beschrieben, gereinigt.

### Reinigung von humanem Vitronectinrezeptor (αᵥβ₃)

Humaner Vitronectinrezeptor wird aus der menschlichen Plazenta nach der Methode von Pytela et al., Methods Enzymol. 1987, 14.4, 475 gewonnen. Humaner Vitronectinrezeptor αᵥβ₃ kann auch aus einigen Zellinien (zum Beispiel aus 293 Zellen, einer humanen embryonalen Nierenzellinie), die mit DNA Sequenzen für beide Untereinheiten αᵥ und β₃ des Vitronectinrezeptors co-transfziert sind, gewonnen werden. Die Untereinheiten wurden mit Octylglycosid extrahiert und anschließend über Concanavalin A, Heparin-Sepharose und S-300 chromatographiert.

### Monoklonale Antikörper

Murine monoklonale Antikörper, spezifisch für die β₃ Untereinheit des Vitronectinrezeptors, werden nach der Methode von Newman et al., Blood, 1985, 227-232, oder nach einem ähnlichen Verfahren hergestellt. Das Kaninchen Fab 2 anti-Maus Fc Konjugat an Meerrettich-Peroxidase (anti-Maus Fc HRP) wurde von Pel Freeze (Katalog Nr. 715 305-1) bezogen.

### ELISA-Test

Die Fähigkeit von Substanzen die Bindung von Kistrin an den Vitronectinrezeptor zu inhibieren, kann mit einem ELISA-Test ermittelt werden. Zu diesem Zweck werden Nunc 96 well-Mikrotiterplatten mit einer Lösung von Kistrin (0.002 mg/ml) nach der Methode von Dennis et al., wie in PROTEINS: Structure, Function and Genetics 1993, 15, 312-321, beschrieben, belegt. Die Platten werden dann zweimal mit PBS/0.05 % Tween-20 gewaschen und durch Inkubieren (60 min) mit RinderserumAlbumin (BSA, 0.5 %, RIA Güteklasse oder besser) in Tris-HCl (50 mM), NaCl (100 mM), MgCl₂ (1 mM), CaCl₂ (1 mM), MnCl₂ (1 mM), pH 7, geblockt. Man stellt Lösungen von bekannten Inhibitoren und von den Testsubstanzen in Konzentrationen von 2 x 10⁻¹² bis 2 x 10⁻⁶ mol/l in Assay-Puffer (BSA (0.5 %, RIA-Güteklasse oder besser) in Tris-HCl (50 mM), NaCl (100 mM), MgCl₂ (1 mM), CaCl₂ (1 mM), MnCl₂ (1 mM), pH 7) her. Die geblockten Platten werden entleert, und jeweils 0.025 ml dieser Lösung, die eine definierte Konzentration (2 x 10⁻¹² bis 2 x 10⁻⁶ mol/l) entweder an einem bekannten Inhibitor oder an einer Testsubstanz enthält, werden in jedes well gegeben. 0.025 ml einer Lösung des Vitronectinrezeptors im Testpuffer (0.03 mg/ml) werden in jedes well der Platte pipettiert und die Platte wird auf einem Schüttler 60-180 min bei Raumtemperatur inkubiert. In der Zwischenzeit wird eine Lösung (6 ml/Platte) eines für die β₃-Untereinheit des Vitronectinrezeptors spezifischen murinen monoklonalen Antikörpers im Assay-Puffer (0.0015 mg/ml) hergestellt. Zu dieser Lösung gibt man einen zweiten Kaninchen-Antikörper (0.001 ml Stammlösung/6 ml der murinen monoklonalen anti-β₃-Antikörper-Lösung), der ein anti-Maus Fc HRP Antikörper Konjugat darstellt, und dieses Gemisch aus murinem anti-β₃ Antikörper und Kaninchen anti-Maus Fc HRP Antikörper Konjugat läßt man während der Zeit der Rezeptor-Inhibitor-Inkubation inkubieren. Die Testplatten werden viermal mit PBS-Lösung, die 0.05 % Tween-20 enthält, gewaschen und man pipettiert jeweils 0.05 ml/well der Antikörpermischung in jedes well der Platte und inkubiert 60-180 min. Die Platte wird viermal mit PBS/0.05 % Tween-20 gewaschen und anschließend mit 0.05 ml/well einer PBS-Lösung, die 0.67 mg/ml o-Phenylendiamin und 0.012 % H₂O₂ enthält, entwickelt. Alternativ hierzu kann o-Phenylendiamin in einem Puffer (pH 5) eingesetzt werden, der Na₃PO₄ und Zitronensäure enthält. Die Farbentwicklung wird mit 1 N H₂SO₄ (0.05 ml/well) gestoppt. Die Absorption wird für jedes well bei 492-405 nm gemessen und die Daten werden nach Standardmethoden ausgewertet.

Es wurden folgende Testergebnisse erhalten:

| Verbindung des Beispiels | K/VnR Inhibierung bei 10 µM (in %) | K/VnR IC₅₀ (µM) |
|---|---|---|
| 1 | 75 | 1.1 |
| 2 | 80 | 0.7 |
| 3 | 77 | 2.2 |
| 4 | 93 | 0.15 |
| 12 | 86 | 0.58 |
| 13 | 92 | 0.19 |
| 14 | 84 | 0.65 |
| 14c | 22 | |
| 15 | 92 | 0.21 |
| 16 | 85 | 0.54 |
| 16b | 29 | |
| 17 | 92 | 0.17 |
| 18 | 95 | 0.075 |
| 19 | 97 | 0.004 |
| 23 | 93 | 0.16 |
| 24 | 95 | 0.052 |
| 25 | 89 | 0.345 |
| 26 | 91 | 0.36 |

## Patentansprüche

1. Verbindungen der Formeln I, 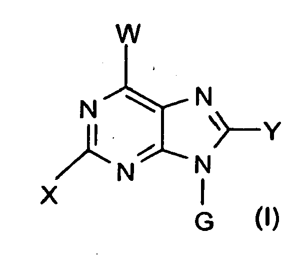 worin bedeuten:
X Wasserstoff;
Y Wasserstoff;
G einen Rest der Formel II
-(CR¹R²)ₙ-A-(CR¹R²)ₘ-(CR¹R³)ᵢ-(CR₁R₂)_{q}-R⁴ (II);
W einen Rest der Formel III
-B-(CR¹R²)ᵣ-A'-(CR¹R²)ₛ-(CR¹R³)ₖ-(CR¹R²)ᵣ-D-E (III);
A, A' eine direkte Bindung;
R¹, R² unabhängig voneinander Wasserstoff oder (C₁-C₂)-Alkyl;
R³ R⁶R^{6'}N-R⁷, R⁶OC(O)N(R⁵)R⁷, R⁶SO₂N(R⁵)R⁷, R⁶C(O)N(R⁵)R⁷ oder R⁶N(R^{6'})C(O)N(R⁵)R⁷;
R⁴ C(O)R⁸;
R⁵ Wasserstoff oder (C₁-C₂)-Alkyl;
R⁶, R^{6'} unabhängig voneinander Wasserstoff, (C₁-C₁₈)-Alkyl, (C₃-C₁₂)-Cycloalkyl, (C₃-C₁₂)-Cycloalkyl-(C₁-C₈)-alkyl, (C₅-C₁₄)-Aryl, worin 1 bis 3 Kohlenstoffatome durch 1 bis 3 Heteroatome aus der Reihe N, S, O ersetzt sein können, oder (C₅-C₁₄)-Aryl-(C₁-C₈)-alkyl, worin in Arylresten 1 bis 3 Kohlenstoffatome durch 1 bis 3 Heteroatome aus der Reihe N, S, O ersetzt sein können, und wobei auch R⁶ und R^{6'} gemeinsam mit den sie verbindenden Atomen ein Ringsystem bilden können, das gegebenenfalls auch zusätzliche Heteroatome aus der Reihe N, S, O enthalten kann;
R7 eine direkte Bindung;
R⁸ Hydroxy, (C₁-C₄)-Alkoxy, (C₅-C₁₄)-Aryl-(C₁-C₄)-alkoxy, (C₅-C₁₄)-Aryloxy, (C₁-C₈)-Alkylcarbonyloxy-(C₁-C₄)-alkoxy oder (C₅-C₁₄)-Aryl-(C₁-C₄)-alkylcarbonyloxy-(C₁-C₄)-alkoxy;
B. 1,4-Pipendindiyl, wobei das Stickstoffatom des Piperidins an das Puringerüst gebunden ist;
D -NR⁶- oder -C(O)-NR⁶-, wobei in der Gruppe -C(O)-NR⁶- das Stickstoffatom an die Gruppe E gebunden ist;
E R⁶R^{6'}N-C(=NR^{6'})- oder einen Rest aus der Reihe 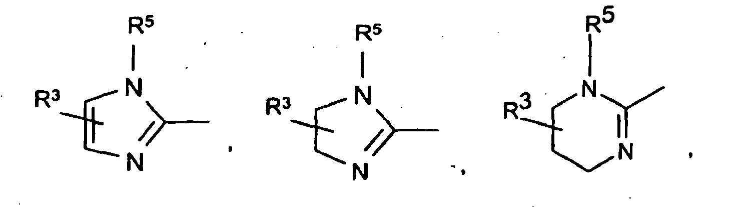 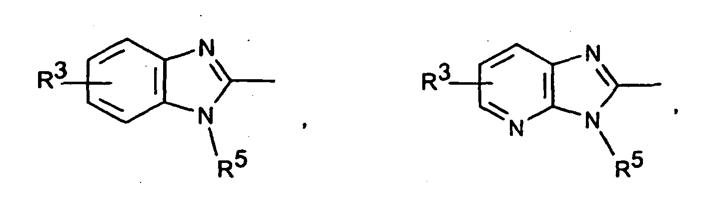
der gegebenenfalls einfach bis dreifach durch Reste aus der Reihe R³, R⁵, =O, =S und R⁶R^{6'}N-C(=NR⁶)- substituiert sein kann;
r null oder eins;
s null;
t null;
k null;
n eins;
m null;
i eins;
q null;
in allen ihren stereoisomeren Formen und Mischungen davon in allen Verhältnissen, und ihre physiologisch verträglichen Salze.

2. Verbindungen der Formel Ih, 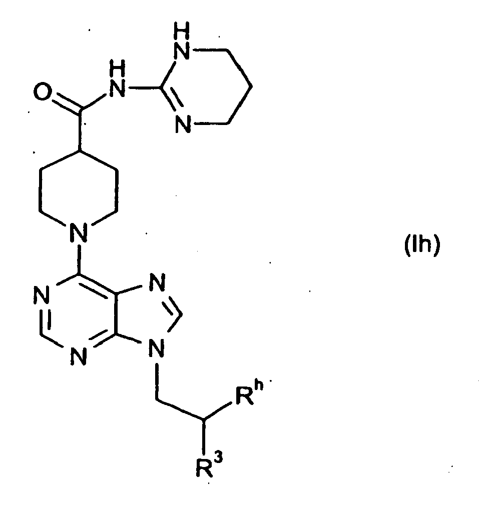 worin R³ für R⁶R^{6'}N-R⁷, R⁶OC(O)N(R⁵)R⁷, R⁶SO₂N(R⁵)R⁷, R⁶C(O)N(R⁵)R⁷ oder R⁶N(R^{6'})C(O)N(R⁵)R⁷ steht und R^{h} für die Carbonsäuregruppe COOH oder für ein Carbonsäurederivat steht; in allen ihren stereoisomeren Formen und Mischungen davon in allen Verhältnissen, und ihre physiologisch verträglichen Salze.

3. 2S-Benzyloxycarbonylamino-3-(6(4-(1,4,5,6-tetrahydropyrimidin-2-yl-arbamoyl)-piperidin-1-yl)-purin-9-yl)-propionsäure und ihre physiologisch verträglichen Salze.

4. Verfahren zur Herstellung einer Verbindung der Formeln I gemäß einem oder mehreren der Ansprüche 1 bis 3, **dadurch gekennzeichnet, daß** man zwei oder mehrere Fragmente, die sich retrosynthetisch aus den Formeln I ableiten lassen, verknüpft.

5. Verfahren gemäß Anspruch 4, **dadurch gekennzeichnet, daß** man zur Herstellung einer Verbindung der Formel I eine Verbindung der Formel IV, 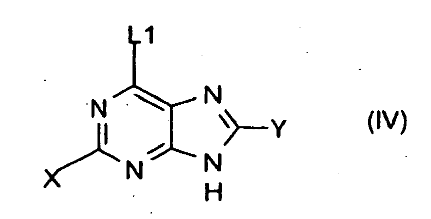 worin L1 für eine Abgangsgruppe steht und X und Y wie in den Ansprüchen 1 bis 3 definiert sind, aber funktionelle Gruppen auch in Form von Vorstufen oder in geschützter Form vorliegen können, schrittweise mit einer Verbindung der Formel V
L2-(CR¹R²)ₙ-A-(CR¹R²)ₘ-(CR¹R³)ᵢ-(CR¹R²)_{q}-R¹⁰ (V)
und mit einer Verbindung der Formel VII
H-B-(CR¹R²)ᵣ-A'-(CR¹R²)ₛ-(CR¹R³)ₖ-(CR¹R²)ᵣ-R¹³ (VII)
zu einer Verbindung der Formel VIII 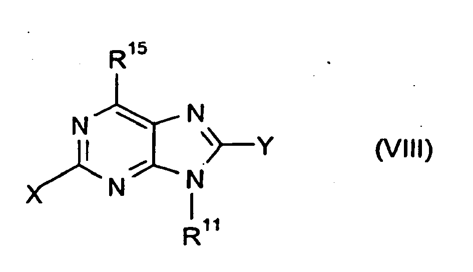 umsetzt, wobei in den Formeln V, VII und VIII
L2 für eine Abgangsgruppe steht;
der Rest R¹¹- für -(CR¹R²)ₙ-A-(CR¹R²)ₘ-(CR¹R³)ᵣ-(CR¹R²)_{q}-R¹⁰ steht;
der Rest R¹⁵- für -B-(CR¹R²)ᵣ-A'-(CR¹R²)ₛ-(CR¹R³)ₖ-(CR¹R²)ₜ-R¹³ steht;
R¹⁰ die in Anspruch 1 angegebenen Bedeutungen von R⁴ hat, wobei die für R⁴ stehenden Gruppen auch in geschützter Form vorliegen können;
R¹³ die in Anspruch 1 angegebenen Bedeutungen der Gruppe D-E hat, wobei in D-E enthaltene Gruppen auch in geschützter Form vorliegen können, oder R¹³ für eine Gruppe steht, die in die Gruppe -D-E umgewandelt werden kann;
R¹, R², R³, A, A', B, X, Y, n, m, i, q, r, s, k und t die in den Ansprüchen 1 bis 3 angegebenen Bedeutungen haben;
und anschließend gegebenenfalls die Gruppen R¹⁰ und R¹³ in die Gruppen R⁴ und D-E überführt;
oder zur Herstellung einer Verbindung der Formel Ia eine Verbindung der Formel IV schrittweise mit einer Verbindung der Formel IX
L2-(CR¹R²)ᵣ-A'-(CR¹R²)ₛ-(CR¹R³)ₖ-(CR¹R²)ₜ-R¹³ (IX)
und mit einer Verbindung der Formel XI
H-B-(CR¹R²)ₙ-A-(CR¹R²)ₘ-(CR¹R³)ᵣ-(CR¹R²)_{q}-R¹⁰ (XI)
zu einer Verbindung der Formel XII 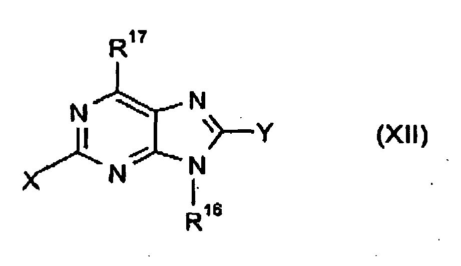 umsetzt, wobei in den Formeln IX, XI und XII
der Rest R¹⁶- für -(CR¹R²)ᵣ-A'-(CR¹R²)ₛ-(CR¹R³)ₖ-(CR¹R²)ᵣ-R¹³ steht;
der Rest R¹⁷- für -B-(CR¹R²)ₙ-A-(CR¹R²)ₘ-(CR¹R³)ᵢ-(CR¹R²)_{q}-R¹⁰ steht;
R¹, R², R³, R¹⁰, R¹³, A, A', B, X, Y, L2, n, m, i, q, r, s, k und t wie für die Formeln V, VII und VIII angegeben definiert sind;
und anschließend gegebenenfalls die Gruppen R¹⁰ und R¹³ in die Gruppen R⁴ und D-E überführt.

6. Verbindungen der Formeln I gemäß einem oder mehreren der Ansprüche 1 bis 3 und/oder ihre physiologisch verträglichen Salze zur Verwendung als Arzneimittel.

7. Verbindungen der Formeln I gemäß einem oder mehreren der Ansprüche 1 bis 3 und/oder ihre physiologisch verträglichen Salze als Inhibitoren der Knochenresorption durch Osteoclasten, als Inhibitoren von Tumorwachstum oder Tumormetastasierung, als Entzündungshemmer, zur Therapie oder Prophylaxe von cardiovaskulären Erkrankungen, zur Therapie oder Prophylaxe von Nephropathien oder Retinopathien, oder als Vitronectinrezeptor-Antagonisten zur Therapie oder Prophylaxe von Krankheiten, die auf der Weohselwirkung zwischen Vitronectinrezeptoren und deren Liganden bei Zell-Zell- oder Zell-Matrix-Interaktionsprozessen beruhen.

8. Pharmazeutisches Präparat, enthaltend mindestens eine Verbindung der Formeln I gemäß einem oder mehreren der Ansprüche 1 bis 3 und/oder ihre physiologisch verträglichen Salze neben pharmazeutisch einwandfreien Trägerstoffen und/oder Zusatzstoffen.

## Claims

1. A compound of the formula I, 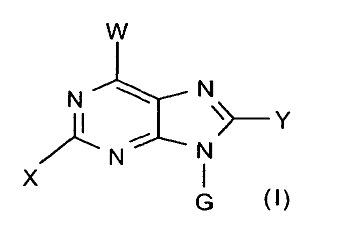
X is hydrogen;
Y is hydrogen;
G is a radical of the formula II
-(CR¹R²)ₙ-A-(CR¹R²)ₘ-(CR¹R³)ᵢ-(CR¹R²)_{q}-R⁴ (II);
W is a radical of the formula III
-B-(CR¹R²)ᵣ-A'-(CR¹R²)ₛ-(CR¹R³)ₖ-(CR¹R²)ₜ-D-E (III);
A, A' is a direct bond;
R¹, R² independently of one another are hydrogen or (C₁-C₂)alkyl;
R³ is R⁶R^{6'}N-R⁷, R⁶OC(O)N(R⁵)R⁷, R⁶SO₂N(R⁵)R⁷, R⁶C(O)N(R⁵)R⁷ or R⁶N(R^{6'})C(O)N(R⁵)R⁷;
R⁴ is C(O)R⁸;
R⁵ is hydrogen or (C₁-C₂)alkyl;
R⁶, R^{6'} independently of one another are hydrogen, (C₁-C₁₈)alkyl, (C₃-C₁₂)cycloalkyl, (C₃-C₁₂)cycloalkyl(C₁-C₈)alkyl, (C₅-C₁₄)-aryl, in which 1 to 3 carbon atoms can be replaced by 1 to 3 heteroatoms from the group consisting of N, S, 0, or (C₅-C₁₄)aryl(C₁-C₈)alkyl, in which 1 to 3 carbon atoms in aryl radicals can be replaced by 1 to 3 heteroatoms from the group consisting of N, S, 0, and it also being possible for R⁶ and R^{6'}, together with the atoms connecting them, to form a ring system which can optionally also contain additional heteroatoms from the group consisting of N, S, 0;
R⁷ is a direct bond;
R⁸ is hydroxyl, (C₁-C₄)alkoxy, (C₅-C₁₄)aryl(C₁-C₄)alkoxy, (C₅-C₁₄)-aryloxy, (C₁-C₈)alkylcarbonyloxy(C₁-C₄)alkoxy or (C₅-C₁₄)aryl(C₁-C₄)alkylcarbonyloxy(C₁-C₄)alkoxy;
B is 1,4-piperidinediyl, the nitrogen atom of the piperidine being bonded to the purine structure;
D is -NR⁶- or -C(O)-NR⁶-, the nitrogen atom in the group -C(O)-NR⁶- being bonded to the group E;
E is R⁶R^{6'}N-C(=NR^{6'})- or a radical from the group consisting of 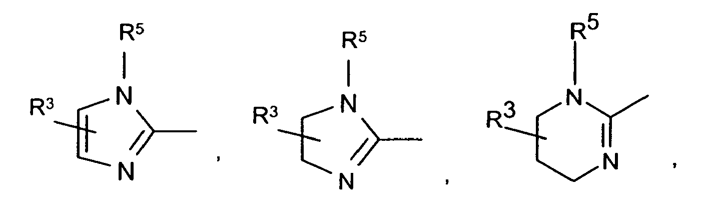 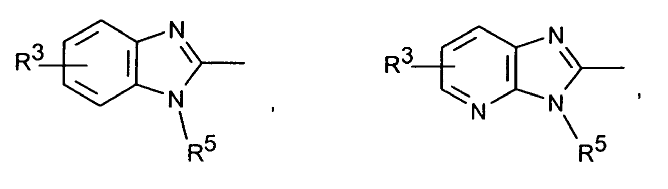
which can optionally be monosubstituted to trisubstituted by radicals from the group consisting of R³, R⁵, =0, =S and R⁶R^{6'}N-C(=NR⁶)-;
r is zero or one;
s is zero;
t is zero;
k is zero;
n is one;
m is zero;
i is one;
q is zero;
in all its stereoisomeric forms and mixtures thereof in all ratios, and its physiologically tolerable salts.

2. A compound of the formula Ih 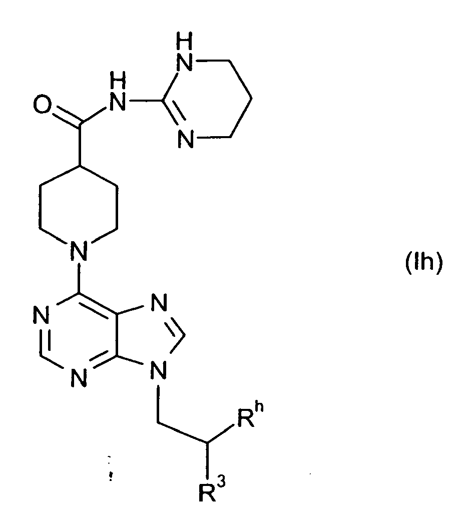 in which R³ is R⁶R^{6'}N-R⁷, R⁶OC(O)N(R⁵)R⁷, R⁶SO₂N(R⁵)R⁷, R⁶C(O)N(R⁵)R⁷ or R⁶N(R^{6'})C(O)N(R⁵)R⁷ and R^{h} is the carboxylic acid group COOH or a carboxylic acid derivative; in all its stereoisomeric forms and mixtures thereof in all ratios, or its physiologically tolerable salts.

3. 2S-Benzyloxycarbonylamino-3-(6-(4-(1,4,5,6-tetrahydropyrimidin-2-yl-carbamoyl)piperidin-1-yl)purin-9-yl)propionic acid or its physiologically tolerable salts.

4. A process for the preparation of a compound of the formula I as claimed in one or more of claims 1 to 3, which comprises linking two or more fragments which can be derived retrosynthetically from the formula I.

5. The process as claimed in claim 4, wherein, for the preparation of a compound of the formula I, a compound of the formula IV 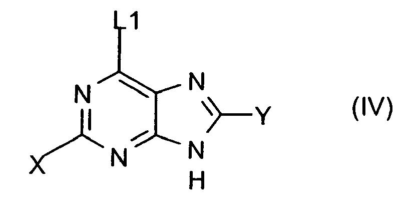 in which L1 is a leaving group and X and Y are as defined in claims 1 to 3, but functional groups can also be present in the form of precursors or in protected form, is reacted stepwise with a compound of the formula V
L2-(CR¹R²)ₙ-A-(CR¹R²)ₘ,-(CR¹R³)ᵢ-(CR¹R²)_{q}-R¹⁰ (V)
and with a compound of the formula VII
H-B-(CR¹R²)ᵣ-A'-(CR¹R²)ₛ-(CR¹R³)ₖ-(CR¹R²)ₜ-R¹³ (VII)
to give a compound of the formula VIII 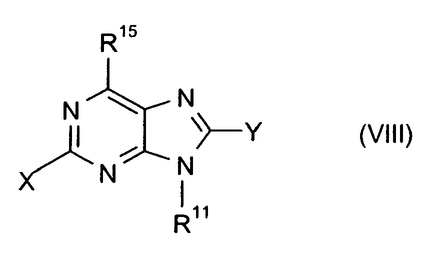 where in the formulae V, VII and VIII
L2 is a leaving group;
the radical R¹¹- is -(CR¹R²)ₙ-A-(CR¹R²)ₘ-(CR¹R³)ᵢ-(CR¹R²)_{q}-R¹⁰;
the radical R¹⁵- is -B-(CR¹R²)ᵣ-A'-(CR¹R²)ₛ-(CR¹R³)ₖ-(CR¹R²)ₜ-R¹³;
R¹⁰ has the meanings of R⁴ indicated in claim 1, where the groups representing R⁴ can also be present in protected form;
R¹³ has the meanings of the group D-E indicated in claim 1, where groups contained in D-E can also be present in protected form, or R¹³ is a group which can be converted into the group -D-E;
R¹, R², R³, A, A', B, X, Y, n, m, i, q, r, s, k and t have the meanings indicated in claims 1 to 3;
and then, if desired, the groups R¹⁰ and R¹³ are converted into the groups R⁴ and D-E;
or, for the preparation of a compound of the formula la, a compound of the formula IV is reacted stepwise with a compound of the formula IX
L2-(CR¹R²)ᵣ-A'-(CR¹R²)ₛ-(CR¹R³)ₖ-(CR¹R²)ₜ-R¹³ (IX)
and with a compound of the formula XI
H-B-(CR¹R²)ₙ-A-(CR¹R²)ₘ-(CR¹R³)_{¡}-(CR¹R²)_{q}-R¹⁰ (XI)
to give a compound of the formula XII 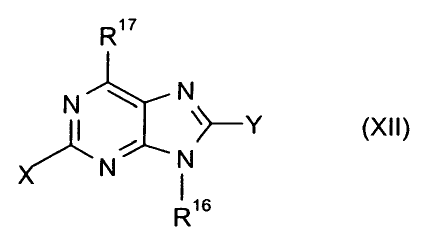 where in the formulae IX, XI and XII
the radical R¹⁶- is -(CR¹R²)ᵣ-A'-(CR¹R²)ₛ-(CR¹R³)ₖ-(CR¹R²)ᵣ-R¹³;
the radical R¹⁷- is -B-(CR¹R²)ₙ-A-(CR¹R²)ₘ-(CR¹R³)ᵢ-(CR¹R²)_{q}-R¹⁰;
R¹, R², R³, R¹⁰, R¹³, A, A', B, X, Y, L2, n, m, i, q, r, s, k and t are defined as indicated for the formulae V, VII and VIII;
and then, if desired, the groups R¹⁰ and R¹³ are converted into the groups R⁴ and D-E.

6. A compound of the formula I as claimed in one or more of claims 1 to 3 and/or its physiologically tolerable salts for use as a pharmaceutical.

7. A compound of the formula I as claimed in one or more of claims 1 to 3 and/or its physiologically tolerable salts as an inhibitor of bone resorption by osteoclasts, as an inhibitor of tumor growth or tumor metastasis, as an antiinflammatory, for the therapy or prophylaxis of cardiovascular disorders, for the therapy or prophylaxis of nephropathies or retinopathies, or as a vitronectin receptor antagonist for the therapy or prophylaxis of illnesses which are based on the interaction between vitronectin receptors and their ligands in cell-cell or cell-matrix interaction processes.

8. A pharmaceutical preparation comprising at least one compound of the formula I as claimed in one or more of claims 1 to 3 and/or its physiologically tolerable salts in addition to pharmaceutically innocuous excipients and/or additives.

## Revendications

1. Composés de formule I 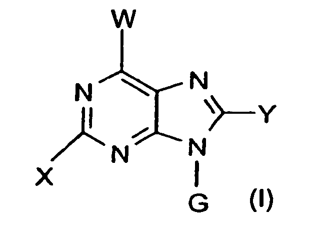 dans laquelle
X représente un atome d'hydrogène ;
Y représente un atome d'hydrogène ;
G représente un groupe de formule II
-(CR¹R²)ₙ-A-(CR¹R²)ₘ-(CR¹R³)ᵢ-(CR¹R²)_{q}-R⁴ (II);
W représente un groupe de formule III
-B-(CR¹R²)ᵣ-A'-(CR¹R²)ₛ-(CR¹R³)ₖ-(CR¹R²)ₜ-D-E (III);
A, A' représentent une liaison directe ;
R¹, R² représentent, indépendamment l'un de l'autre, un atome d'hydrogène ou un groupe alkyle (en C₁ à C₂) ;
R³ représente R⁶R^{6'}N-R⁷, R⁶OC(O)N(R⁵)R⁷, R⁶SO₂N(R⁵)R⁷, R⁶C(O)N(R⁵)R⁷ ou R⁶N(R^{6'})C(O)N(R⁵)R⁷ ;
R⁴ représente C(O)R⁸ ;
R⁵ représente un atome d'hydrogène ou un groupe alkyle (en C₁ à C₂) ;
R⁶, R^{6'} représentent, indépendamment l'un de l'autre, un atome d'hydrogène, un groupe alkyle (en C₁ à C₁₈), cycloalkyle (en C₃ à C₁₂), cycloalkyle (en C₃ à C₁₂)-alkyle (en C₁ à C₈), aryle (en C₅ à C₁₄), où 1 à 3 atomes de carbone peuvent être substitués par 1 à 3 hétéroatomes de la série N, S, O, ou aryle (en C₅ à C₁₄)-alkyle (en C₁ à C₈), où, dans les groupes aryle, 1 à 3 atomes de carbone peuvent être substitués par 1 à 3 hétéroatomes de la série N, S, O, et R⁶ et R^{6'} peuvent former, conjointement avec les atomes qui les relient, un système cyclique qui peut éventuellement contenir des hétéroatomes supplémentaires de la série N, S, O ;
R⁷ représente une liaison directe ;
R⁸ représente un groupe hydroxy, alcoxy (en C₁ à C₄), aryle (en C₅ à C₁₄)-alcoxy (en C₁ à C₄), aryloxy (en C₅ à C₁₄), alkyle (en C₁ à C₈)-carbonyloxy-alcoxy (en C₁ à C₄) ou aryle (en C₅ à C₁₄)-alkyle (en C₁ à C₄)-carbonyloxy-alcoxy (en C₁ à C₄) ;
B représente un groupe 1,4-pipéridinediyle, où l'atome d'azote de la pipéridine est lié au squelette purine ;
D représente un groupe -NR⁶- ou -C(O)-NR⁶- où, dans le groupe -C(O)-NR⁶-, l'atome d'azote est lié au groupe E ;
E représente un groupe R⁶R^{6'}N-C(=NR^{6'})- ou un groupe de la série 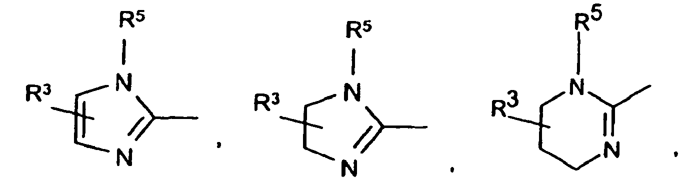 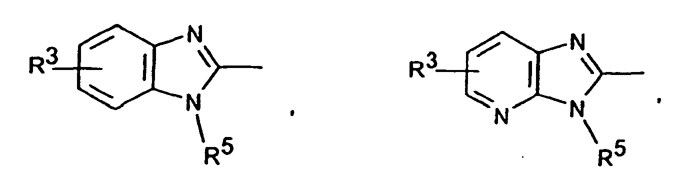
qui peut éventuellement être substitué une à trois fois par des groupes de la série R³, R⁵, =0, =S et R⁶R^{6'}N-C(=NR⁶)- ;
r vaut zéro ou un ;
s vaut zéro ;
t vaut zéro ;
k vaut zéro ;
nvaut un ;
m vaut zéro ;
i vaut un ;
q vaut zéro ;
dans toutes leurs formes stéréoisomères et tous les mélanges de celles-ci à tout rapport, et leurs sels acceptables sur le plan physiologique.

2. Composés de formule Ih, 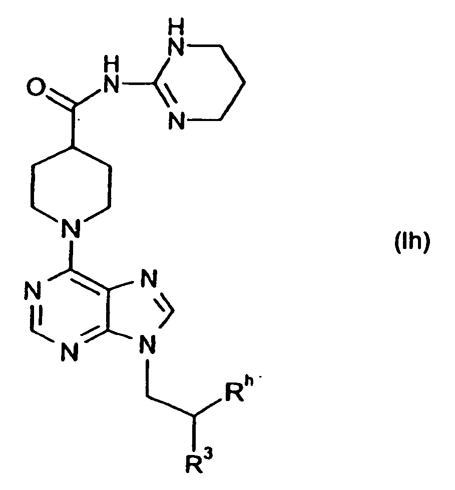 dans laquelle R³ représente R⁶R^{6'}N-R⁷, R⁶OC(O)N(R⁵)R⁷, R⁶SO₂N(R⁵)R⁷, R⁶C(O)N(R⁵)R⁷ ou R⁶N(R^{6'}(O)N(R⁵)R⁷ et R^{h} représente le groupe acide carboxylique COOH ou un dérivé d'acide carboxylique ; dans toutes leurs formes stéréoisomères et tous les mélanges de celles-ci à tout rapport, et leurs sels acceptables sur le plan physiologique.

3. Acide 2S-benzyloxycarbonylamino-3-(6-(4-(1,4,5,6-tétrahydro-pyrimidine-2-yl-carbamoyl)-pipéridine-1-yl)-purine-9-yl)-propionique et ses sels acceptables sur le plan physiologique.

4. Procédé de préparation d'un composé de formule I selon l'une quelconque ou plusieurs des revendications 1 à 3, **caractérisé en ce que** l'on assemble deux fragments ou plus, qui peuvent être dérivés par rétrosynthèse des formules I.

5. Procédé selon la revendication 4, **caractérisé en ce que**, pour la préparation d'un composé de formule I, un composé de formule IV, 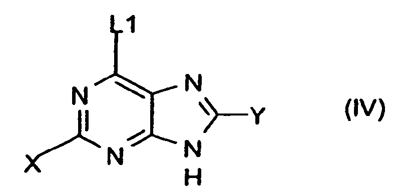 dans laquelle L1 représente un groupe partant et X et Y sont tels que définis dans les revendications 1 à 3, mais des groupes fonctionnels peuvent également se présenter sous la forme de précurseurs ou sous une forme protégée, est mis à réagir progressivement avec un composé de formule V
L2-(CR¹R²)ₙ-A-(CR¹R²)ₘ-(CR¹R³)ᵢ-(CR¹R²)_{q}-R¹⁰ (V)
et avec un composé de formule VII
H-B-(CR¹R²)ᵣ-A'(CR¹R²)ₛ-(CR¹R³)ₖ-(CR¹R²)ₜ-R¹³ (VII)
pour former un composé de formule VIII 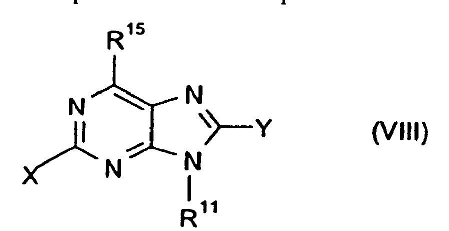 où, dans les formules V, VII et VIII,
L2 représente un groupe partant ;
le groupe R¹¹- représente -(CR¹R²)ₙ-A-(CR¹R²)ₘ-(CR¹R³)ᵢ-(CR¹R²)_{q}-R¹⁰ ;
le groupe R¹⁵- représente -B-(CR¹R²)ᵣ-A'-(CR¹R²)ₛ-(CR¹R³)ₖ-(CR¹R²)ₜ-R¹³ ;
R¹⁰ a les significations mentionnées dans la revendication 1 de R⁴, les groupes mentionnés pour R⁴ pouvant également se présenter sous une forme protégée ;
R¹³ a les significations mentionnées dans la revendication 1 du groupe D-E, les groupes contenus dans D-E pouvant également se présenter sous une forme protégée, ou R¹³ représente un groupe qui peut être converti en groupe -D-E ;
R¹, R², R³, A, A', B, X, Y, n, m, i, q, r, s, k et t ont les significations mentionnées dans les revendications 1 à 3 ;
et on convertit éventuellement ensuite les groupes R¹⁰ et R¹³ en groupes R⁴ et D-E ;
ou, pour la préparation d'un composé de formule Ia, on fait réagir progressivement un composé de formule IV avec un composé de formule IX
L2-(CR¹R²)ᵣ-A'-(CR¹R²)ₛ-(CR¹R³)ₖ-(CR¹R²)ₜ-R¹³ (IX)
et avec un composé de formule XI
H-B-(CR¹R²)ₙ-A-(CR¹R²)ₘ-(CR¹R³)ᵢ-(CR¹R²)_{q}-R¹⁰ (XI)
pour former un composé de formule XII 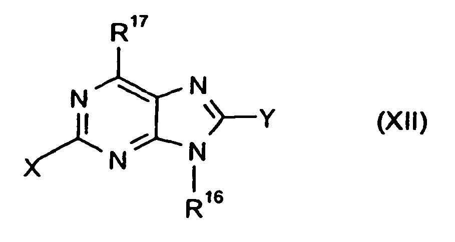 où, dans les formules IX, XI et XII
le groupe R¹⁶- représente -(CR¹R²)ᵣ-A'-(CR¹R²)ₛ-(CR¹R³)ₖ-(CR¹R²)ₜ-R¹³ ;
le groupe R¹⁷- représente -B-(CR¹R²)ₙ-A-(CR¹R²)ₘ-(CR¹R³)ᵢ-(CR¹R²)_{q}-R¹⁰ ;
R¹, R², R³, R¹⁰, R¹³, A, A', B, X, Y, L2, n, m, i, q, r, s, k et t sont tels que définis dans les formules V, VII et VIII ;
et on convertit éventuellement ensuite les groupes R¹⁰ et R¹³ en groupes R⁴ et D-E.

6. Composés de formule I selon l'une quelconque ou plusieurs des revendications 1 à 3 et/ou leurs sels acceptables sur le plan physiologique destinés à être utilisés en tant que médicaments.

7. Composés de formule I selon l'une quelconque ou plusieurs des revendications 1 à 3 et/ou leurs sels acceptables sur le plan physiologique utilisés en tant qu'inhibiteurs de la résorption osseuse par des ostéoclastes, en tant qu'inhibiteurs de la croissance tumorale ou de la métastase tumorale, en tant qu'anti-inflammatoires, pour la thérapie ou la prophylaxie de maladies cardiovasculaires, pour la thérapie ou la prophylaxie de néphropathies ou de rétinopathies, ou en tant qu'antagonistes des récepteurs de la vitronectine pour la thérapie ou la prophylaxie de maladies qui reposent sur l'interaction entre les récepteurs de la vitronectine et leurs ligands dans des processus d'interaction cellule-cellule ou cellule-matrice.

8. Préparation pharmaceutique, contenant au moins un composé de formule I selon l'une quelconque ou plusieurs des revendications 1 à 3 et/ou ses sels acceptables sur le plan physiologique en plus de véhicules et/ou d'adjuvants acceptables sur le plan pharmaceutique.
